# EUROPÄISCHE PATENTSCHRIFT

(11) **EP 2 954 326 B1**
(45) Veröffentlichungstag und Bekanntmachung des Hinweises auf die Patenterteilung: **19.07.2017**
(21) Anmeldenummer: 14703064.7
(22) Anmeldetag: 04.02.2014
(51) Int. Cl.: G01N 33/569

(54) **VERFAHREN UND VORRICHTUNG ZUM UNTERSUCHEN EINER ZELLPROBE**
METHOD AND DEVICE FOR EXAMINING A CELL SAMPLE
PROCÉDÉ ET DISPOSITIF POUR L'ÉTUDE D'UN ÉCHANTILLON DE CELLULE

(30) Priorität: 05.02.2013 DE 102013101138
(43) Veröffentlichungstag der Anmeldung: 16.12.2015
(73) Patentinhaber: Toposnomos Ltd., 81373 München (DE)
(72) Erfinder: SCHUBERT, Walter, 53783 Eitorf (DE)
(74) Vertreter: Hofstetter, Schurack & Partner
(86) Internationale Anmeldenummer: PCT/EP2014/052168
(87) Internationale Veröffentlichungsnummer: WO 2014/122133

(56) Entgegenhaltungen:
- SCHUBERT WALTER: "TOPOLOGICAL PROTEOMICS, TOPONOMICS, MELK-TECHNOLOGY", ADVANCES IN BIOCHEMICAL ENGINEERING, BIOTECHNOLOGY, SPRINGER, BERLIN, DE, Bd. 83, 1. Januar 2003 (2003-01-01), Seiten 189-209, XP008079848, ISSN: 0724-6145
- SCHUBERT WALTER ET AL: "Analyzing proteome topology and function by automated multidimensional fluorescence microscopy", NATURE BIOTECHNOLOGY, NATURE PUBLISHING GROUP, NEW YORK, NY, US, Bd. 24, Nr. 10, 1. Oktober 2006 (2006-10-01), Seiten 1270-1278, XP002438060, ISSN: 1087-0156, DOI: 10.1038/NBT1250
- BERNDT UTA ET AL: "Proteomic analysis of the inflamed intestinal mucosa reveals distinctive immune response profiles in Crohn's disease and ulcerative collitis", JOURNAL OF IMMUNOLOGY, Bd. 179, Nr. 1, Juli 2007 (2007-07), Seiten 295-304, XP002721811, ISSN: 0022-1767
- UTA BERNDT ET AL: "Systematic high-content proteomic analysis reveals substantial immunologic changes in colorectal cancer", CANCER RESEARCH, AMERICAN ASSOCIATION FOR CANCER RESEARCH, US, Bd. 68, Nr. 3, 1. Februar 2008 (2008-02-01), Seiten 880-888, XP002618323, ISSN: 0008-5472, DOI: 10.1158/0008-5472.CAN-07-2923

## Beschreibung

Die Erfindung betrifft ein Verfahren und eine Vorrichtung zum Untersuchen einer Zellprobe.

Die stetig zunehmende Ineffizienz in der Behandlung chronischer Krankheiten - trotz eleganter und logischer molekularbiologischer Studien und hilfreicher angewandter Mathematik - hat in letzter Zeit zu dringenden Warnungen geführt (World Alzheimers Report 2011). Die übergreifende Fragestellung ernstlich besorgter Wissenschaftler und Redakteure lautet "Where are we going wrong" (Was machen wir falsch), und ähnliche Bedenken wurden von anderen formuliert, während die desillusionierte Pharmaindustrie Untersuchungseinrichtungen schließt und tausende von Angestellten entlässt. Überraschenderweise wurden entsprechende frühe Warnungen in einem Bericht mit dem Titel "The Fruits of Genomics" (Lehman Brothers und Mc Kinsey 2001) von der Wissenschaft völlig in Abrede gestellt, derweil der eigentliche Grund des Problems offensichtlich noch gar nicht bekannt ist, und viele Bedenken hinsichtlich der gegenwärtigen Entwicklung zu hohen Ausfallquoten von Medikamenten zur Behandlung von Krebserkrankungen und zu Fehlschlägen bei der Behandlung von Alzheimererkrankungen sind immer noch ohne klares Lösungskonzept. Offensichtlich sind die vielen eleganten, logischen molekular- und zellbiologischen Studien und Modellstudien mit ihren überzeugenden wissenschaftlichen Erklärungen gescheitert bei der Übertragung der entsprechenden Konzepte auf Alzheimer-Therapien, und ähnliche Erfahrungen wurden im Bereich der Krebsforschung gemacht. Dies weist darauf hin, dass die Logik der aktuellen wissenschaftlichen Praxis mit ihren etablierten zellbiologischen und tierischen Modellen nicht mit der Logik der chronischen Krankheit als solchen übereinstimmt.

In der Publikation Schubert W: "Topological proteomics, toponomics, MELKtechnology" (Adv Biochem Eng Biotechnol. 2003;83:189-209) wird die sogenannte MELK-Technologie (Multi-Epitop-Ligand-Kartographie) beschrieben, welche die Analyse von Protein-Mustern ermöglicht, wodurch das sogenannte Toponom von Zellen ermittelt werden kann, welches Aufschluss über die zeitliche und örtliche Organisation von miteinander interagierenden Proteinen in Zellen erlaubt.

In der Publikation Walter Schubert et al.: "Analyzing proteome topology and function by automated multidimensional fluorescence microscopy" (Nature Biotechnology 24, 1270-1278 (2006)) wird die Bedeutung des Toponoms, das heißt der zeitlichen und örtlichen Organisation von miteinander interagierenden Proteinen in Zellen diskutiert.

Die Publikation Berndt U. et al.: "Proteomic analysis of the inflamed intestinal mucosa reveals distinctive immune response profiles in Crohn's disease and ulcerative colitis" (J Immunol. 2007 Jul 1;179(1):295-304) befasst sich mit Veränderungen in der Verteilung und Zuordnung von Schleimhautproteinen zur Aufklärung der Pathogenese von Morbus Crohn und Colitis ulcerosa.

Die Publikation Berndt U. et al.: "Systematic high-content proteomic analysis reveals substantial immunologic changes in colorectal cancer" 8Cancer Res. 2008 Feb 1;68(3):880-8) befasst sich mit der Rolle des Immunsystems bei der Entstehung von kolorektalen Karzinomen und verwendet zur Untersuchung ein MELK-Analysesystem.

Aufgabe der vorliegenden Erfindung ist es, ein Verfahren zu schaffen, welches die Ermittlung von Krankheitsmechanismen erlauben.

Die Aufgabe wird erfindungsgemäß durch ein Verfahren mit den Merkmalen des Patentanspruchs 1 gelöst. Vorteilhafte Ausgestaltungen mit zweckmäßigen Weiterbildungen der Erfindung sind in den Unteransprüchen angegeben.

Ein erster Aspekt der Erfindung betrifft ein Verfahren zum Untersuchen einer Zellprobe. Erfindungsgemäß umfasst das Verfahren zumindest die Schritte Ermitteln von ortsaufgelösten kombinatorischen Bindungsmustern (KBM) mit Hilfe einer wenigstens zwei Liganden umfassenden Ligandenbibliothek in wenigstens zwei Zellelementen wenigstens einer Zelle der Zellprobe und Vergleichen der ermittelten KBMs. Unter einer Zellprobe werden im Rahmen der vorliegenden Erfindung insbesondere einzelne oder mehrere Gewebeproben, einzelne oder mehrere Gewebeschnitte, Serienschnitte, einzelne Zellen, mehrere Zellen des gleichen Typs und/oder unterschiedlicher Typen, Zellverbunde sowie Bereiche des Extrazellularraums, insbesondere Extrazellularmatrix, verstanden. Unter einem ortsaufgelösten kombinatorischen Bindungsmuster (KBM) sind im Rahmen der vorliegenden Erfindung solche Muster zu verstehen, bei denen an wenigstens zwei und vorzugsweise an mehreren Orten der Zellprobe ermittelt wird, ob am betrachteten Ort eine strikte Kolokalisierung und/oder eine strikte Anti-Kolokalisierung und/oder eine variable Kolokalisierung bzw. Anti-Kolokalisierung der wenigstens zwei Liganden vorliegt. Die KBMs, welche grundsätzlich auch als CMP (combinatorial molecular patterns) bezeichnet werden können, werden bevorzugt innerhalb vorgegebener Volumen- und/oder Flächenelemente ermittelt. Jedes Volumen- und/oder Flächenelement weist hierzu ein vorgegebenes Volumen bzw. eine vorgegebene Fläche auf. Das jeweilige Volumen bzw. die jeweilige Fläche kann beispielsweise maschinell bzw. automatisch und/oder abhängig von der betrachteten Zellprobe und/oder abhängig vom betrachteten Zellelement und/oder ortsabhängig und/oder abhängig von der jeweiligen Fragestellung und/oder von einem Fachmann vorgegeben werden. Beispielsweise kann das Flächenelement eine Fläche von höchstens 1,5 µm*1,5µm (2,25 (µm²) aufweisen. Weiterhin kann vorgesehen sein, dass das Flächenelement eine Fläche von 2,25 µm², 2,23 µm², 2,21 µm², 2,19 µm², 2,17 µm², 2,15 µm², 2,13 µm², 2,11 µm², 2,09 µm², 2,07 µm², 2,05 µm², 2,03 µm², 2,01 µm², 1,99 µm², 1,97 µm², 1,95 µm², 1,93 µm², 1,91 µm², 1,89 µm², 1,87 µm², 1,85 µm², 1,83 µm², 1,81 µm², 1,79 µm², 1,77 µm², 1,75 µm², 1,73 µm², 1,71 µm², 1,69 µm², 1,67 µm², 1,65 µm², 1,63 µm², 1,61 µm², 1,59 µm², 1,57 µm², 1,55 µm², 1,53 µm², 1,51 µm², 1,49 µm², 1,47 µm², 1,45 µm², 1,43 µm², 1,41 µm², 1,39 µm², 1,37 µm², 1,35 µm², 1,33 µm², 1,31 µm², 1,29 µm², 1,27 µm², 1,25 µm², 1,23 µm², 1,21 µm², 1,19 µm², 1,17 µm², 1,15 µm², 1,13 µm², 1,11 µm², 1,09 µm², 1,07 µm², 1,05 µm², 1,03 µm², 1,01 µm², 0,99 µm², 0,97 µm², 0,95 µm², 0,93 µm², 0,91 µm², 0,89 µm², 0,87 µm², 0,85 µm², 0,83 µm², 0,81 µm², 0,79 µm², 0,77 µm², 0,75 µm², 0,73 µm², 0,71 µm², 0,69 µm², 0,67 µm², 0,65 µm², 0,63 µm², 0,61 µm², 0,59 µm², 0,57 µm², 0,55 µm², 0,53 µm², 0,51 µm², 0,49 µm², 0,47 µm², 0,45 µm², 0,43 µm², 0,41 µm², 0,39 µm², 0,37 µm², 0,35 µm², 0,33 µm², 0,31 µm², 0,29 µm², 0,27 µm², 0,25 µm², 0,23 µm², 0,21 µm², 0,19 µm², 0,17 µm², 0,15 µm², 0,13 µm², 0,11 µm² oder weniger aufweist. Weiterhin kann grundsätzlich vorgesehen sein, dass das Flächenelement eine Fläche von mehr als 2,25 µm² aufweist. Das Flächenelement kann grundsätzlich eine beliebige geometrische Form aufweisen und beispielsweise quadratisch, rechtwinklig, rund, oval, dreieckig, mehreckig oder unregelmäßig gewählt sein. Das Volumenelement kann beispielsweise ein Volumen von höchstens 1,5 µm*1,5µm*1,5 µm (3,375 µm³) aufweisen. Beispielsweise kann das Volumenelement ein Volumen von 3,375 µm³, 3,325 µm³, 3,275 µm³, 3,225 µm³, 3,175 µm³, 3,125 µm³, 3,075 µm³, 3,025 µm³, 2,975 µm³, 2,925 µm³, 2,875 µm³, 2,825 µm³, 2,775 µm³, 2,725 µm³, 2,675 µm³, 2,625 µm³, 2,575 µm³, 2,525 µm³, 2,475 µm³, 2,425 µm³, 2,375 µm³, 2,325 µm³, 2,275 µm³, 2,225 µm³, 2,175 µm³, 2,125 µm³, 2,075 µm³, 2,025 µm³, 1,975 µm³, 1,925 µm³, 1,875 µm³, 1,825 µm³, 1,775 µm³, 1,725 µm³, 1,675 µm³, 1,625 µm³, 1,575 µm³, 1,525 µm³, 1,475 µm³, 1,425 µm³, 1,375 µm³, 1,325 µm³, 1,275 µm³, 1,225 µm³, 1,175 µm³, 1,125 µm³, 1,075 µm³, 1,025 µm³, 0,975 µm³, 0,925 µm³, 0,875 µm³, 0,825 µm³, 0,775 µm³, 0,725 µm³, 0,675 µm³, 0,625 µm³, 0,575 µm³, 0,525 µm³, 0,475 µm³, 0,425 µm³, 0,375 µm³, 0,325 µm³, 0,275 µm³, 0,225 µm³, 0,175 µm³, 0,125 µm³ oder weniger aufweisen. Weiterhin kann vorgesehen sein, dass das Volumenelement ein Volumen von mehr als 3,375 µm³ aufweist. Auch das Volumenelement kann grundsätzlich eine beliebige geometrische Form aufweisen und beispielsweise würfelförmig, kugelförmig, quaderförmig, prismatisch etc. gewählt sein. Biomoleküle werden vorzugsweise dann als kolokalisiert angesehen, wenn sie einen lateralen Abstand von höchstens 500 nm, beispielsweise einen Abstand von 500 nm, 495 nm, 490 nm, 485 nm, 480 nm, 475 nm, 470 nm, 465 nm, 460 nm, 455 nm, 450 nm, 445 nm, 440 nm, 435 nm, 430 nm, 425 nm, 420 nm, 415 nm, 410 nm, 405 nm, 400 nm, 395 nm, 390 nm, 385 nm, 380 nm, 375 nm, 370 nm, 365 nm, 360 nm, 355 nm, 350 nm, 345 nm, 340 nm, 335 nm, 330 nm, 325 nm, 320 nm, 315 nm, 310 nm, 305 nm, 300 nm, 295 nm, 290 nm, 285 nm, 280 nm, 275 nm, 270 nm, 265 nm, 260 nm, 255 nm, 250 nm, 245 nm, 240 nm, 235 nm, 230 nm, 225 nm, 220 nm, 215 nm, 210 nm, 205 nm, 200 nm, 195 nm, 190 nm, 185 nm, 180 nm, 175 nm, 170 nm, 165 nm, 160 nm, 155 nm, 150 nm, 145 nm, 140 nm, 135 nm, 130 nm, 125 nm, 120 nm, 115 nm, 110 nm, 105 nm, 100 nm, 95 nm, 90 nm, 85 nm, 80 nm, 75 nm, 70 nm, 65 nm, 60 nm, 55 nm, 50 nm, 45 nm, 40 nm, 35 nm, 30 nm, 25 nm, 20 nm, 15 nm, 10 nm, 5 nm oder weniger aufweisen. Es kann vorgesehen sein, dass das Volumen- und/oder Flächenelement über die Zellprobe bzw. in Abhängigkeit des betrachteten Zellelements konstant vorgegeben und/oder variiert wird. Für jede Zellprobe werden mindestens zwei KBMs ermittelt und miteinander verglichen. Mit anderen Worten werden eine Kolokalisierung und/oder eine Anti-Kolokalisierung und/oder eine variable Ko- bzw. Antikolokalisierung der wenigstens zwei Liganden, das heißt der von diesen gebundenen Biomoleküle innerhalb von mindestens zwei voneinander verschiedenen Volumen- und/oder Flächenelementen der Zellprobe bzw. der betrachteten Zellelemente ermittelt und miteinander vergleichen. Als Liganden können grundsätzlich alle chemischen Elemente und/oder Verbindungen verwendet werden, die mit biologischen Strukturen bzw. mit Biomolekülen wechselwirken. Die Art und Anzahl der Liganden kann in Abhängigkeit der bereitgestellten Zellprobe und/oder in Abhängigkeit des betrachteten Zellelements und/oder in Abhängigkeit der konkreten Fragestellung gewählt und/oder angepasst werden. Die verwendeten Liganden werden zweckmäßigerweise in einer Liganden- oder Testbibliothek zusammengefasst, wobei sich die Ligandenbibliothek grundsätzlich aus zwei oder mehr Teilligandenbibliotheken zusammensetzen kann. Es kann vorgesehen sein, dass die Ligandenbibliothek und/oder die Teilligandenbibliotheken einen oder mehrere Liganden umfasst bzw. umfassen, die spezifisch oder zumindest weitgehend spezifisch mit einem oder mehreren Bestandteilen eines bestimmten Zellelements wechselwirken. Alternativ oder zusätzlich kann vorgesehen sein, dass die Ligandenbibliothek und/oder die Teilligandenbibliotheken einen oder mehrere Liganden umfasst bzw. umfassen, die unspezifisch oder zumindest weitgehend unspezifisch mit einem bestimmten Zellelement bzw. mit einem bestimmten Bestandteil des betrachteten Zellelements wechselwirken. Weiterhin kann vorgesehen sein, dass die Ligandenbibliothek in Abhängigkeit des Vergleichsergebnisses um weitere Liganden erweitert wird. Ebenso kann es vorgesehen sein, dass ein oder mehrere Liganden der Ligandenbibliothek gegen einen oder mehrere andere Liganden ausgetauscht wird bzw. werden. Durch einen Vergleich der ermittelten KBMs, das heißt indem geprüft wird, ob und falls ja inwieweit sich die KBMs unterschiedlicher Zellelemente unterscheiden, erhält man Informationen über das Vorliegen einer pathologischen Veränderung der betrachteten Zellprobe sowie, falls eine solche pathologische Veränderung vorliegt, über den zellulären Mechanismus dieser pathologischen Veränderung. Dies ermöglicht im Gegensatz zum Stand der Technik die Identifizierung krankheitsspezifischer Mechanismen auf zellulärer Ebene, wodurch erstmals konkrete und eindeutige Angriffspunkte für die Bereitstellung und Verwendung krankheitsspezifischer Medikamente und Behandlungsstrategien erhältlich sind. Die Erfindung beruht dabei auf verschiedenen Erkenntnissen:
Zellfunktionalitäten umfassen mindestens vier Organisationsebenen: Genom, Transkriptom, Proteom und Toponom. Das Toponom beispielsweise ist der räumliche Netzwerkcode von Proteinen und anderen Biomolekülen (z.B. von Kohlehydraten und Nukleinsäuren) in morphologisch intakten Zellen und Geweben. Der Begriff Toponom leitet sich von den altgriechischen Nomen "*TÓΠOζ*" (topos = Ort, Position) und "vóµoζ" (nomos = Gesetz) ab. Es wird durch experimentell gewonnene Erkenntnisse und durch direkte Visualisierung der Toponomstrukturen nachgewiesen. Das Toponom macht deutlich, dass eine dieses biomolekulare Netzwerk organisierende Zelle topologischen Regeln folgt und so koordinierte Wechselwirkungen zwischen seinen molekularen Bestandteilen erlaubt: In einer Zelle oder in der extrazellulären Matrix muss jeder einzelne molekulare Bestandteil einer solchen Wechselwirkung in der richtigen Konzentration zum richtigen Zeitpunkt und am richtigen subzellulären Ort sein, damit ein spezifisches molekulares Netzwerk ausgebildet werden kann. Diese Wechselwirkung kann entweder auf der Grundlage von starken oder schwachen physikalischen Zusammenlagerungen von Biomolekülen stattfinden und ebenso indirekt, mittels diffusionsfähiger Moleküle, die an bestimmten Orten an andere Biomoleküle, wie etwa Proteine binden. Biomolekulare Netzwerke sind also durch einen nicht-zufälligen räumlichen Kontext ihrer molekularen Elemente charakterisiert. Folglich gehorcht jedes eine konkrete Zellfunktionalität ausführende biomolekulare Netzwerk Regeln von topologisch definierten Anordnungen von Biomolekülen - ein Raumcode (Toponom), der eine gerichtete Handlung, wie etwa einen spezifischen Informationsfluss entlang eines Pfads, ermöglicht. Das Toponom enthält den Ausführungskodex, der diese Funktionalitäten anwendet. Toponomics ist eine Disziplin der Systembiologie, Zellbiologie und Histologie und betrifft die Untersuchung des Toponoms von Organismen. Das Projekt zum menschlichen Toponom (human toponome project) umfasst die vollständige Dekodierung des menschlichen Toponoms mit 20.000 unterschiedlichen Proteinen in einer großen Auswahl von Zelltypen, Geweben und Krankheitsbildern. Erfindungsgemäß wird die kombinatorische molekulare Struktur (KBM) großer molekularer Strukturen in situ (in einer Zelle oder einem Gewebe mit intakter Struktur) beurteilt, indem mindestens zwei und vorzugsweise Dutzende, Hunderte oder Tausende verschiedener Proteine und/oder anderer Biomoleküle in mindestens zwei unterschiedlichen subzellulären Strukturen (Zellelementen) morphologisch intakter, fixierter Zell- oder Gewebeabschnitte gleichzeitig kokartiert (co-map) und die sich ergebenden KBMs verglichen werden.

Der Vorteil des erfindungsgemäßen Verfahrens besteht darin, dass Krankheitsmechanismen am Zielort der Krankheit im menschlichen oder tierischen Gewebe unmittelbar entschlüsselt werden können, ohne dass auf indirekte Modell (Mausmodelle, Zellmodelle etc.), die den erwiesenen Nachteil sehr geringer Prädiktivität für den Menschen mit sich bringen (Literatur: Hutchinson L, Kirck R. High drug attrition rates -where are we going wrong? Nat Rev Clin Oncol 2011; 8, 189 - 190), zurückgegriffen werden muss. Darüber hinaus besteht die Möglichkeit, die Gesamtentschlüsselung des Toponoms für bis zu 20000 oder mehr verschiedene menschliche und/oder tierische Proteine/Biomoleküle durch die Verfügbarkeit von humanen und/oder tierischen rekombinanten Antikörpern durchzuführen. Weiterhin kann vorgesehen sein, dass dieses "Toponome decoding" zusammen mit Genome Sequencing an ein und demselben Gewebe durchgeführt wird. Dies erlaubt erstmals die Entschlüsselung biologischer Grundprinzipien sowie die Entschlüsselung des gesamten Funktionsplans biologischer Zellen und Gewebe.

Erfindungsgemäß ist es vorgesehen, dass die KBMs in wenigstens zwei Zellelementen, welche sich zumindest bezüglich ihrer Reihenfolge entlang des biologischen Informationsflusses der Zelle unterscheiden, ermittelt werden und/oder bei welchem die wenigstens zwei KBMs entgegen der Reihenfolge des biologischen Informationsflusses der Zelle ermittelt werden. Unter einem biologischen Informationsfluss sind dabei im Rahmen der Erfindung der Ort und die Richtung der Biogenese, des Transports und des Prozessierens von Biomolekülen (Proteine, Kohlenhydrate, Nukleinsäuren, Fette, Aminosäuren) zu verstehen. In all diesen Biomolekülen ist Information enthalten, die vielfach transportiert und modifiziert werden kann. Indem die KBMs in Zellelementen ermittelt werden, die sich zumindest bezüglich ihrer Reihenfolge entlang des biologischen Informationsflusses der Zelle unterscheiden, können somit durch den Vergleich der KBMs Informationen über den Ort und den Mechanismus etwaiger pathologischer Veränderungen besonders einfach ermittelt werden. Wenn die wenigstens zwei KBMs entgegen der Reihenfolge des biologischen Informationsflusses der Zelle ermittelt werden (Reverse Imaging), können Informationen über den Ort und den Mechanismus etwaiger pathologischer Veränderungen auf besonders einfache und systematische Weise sowie hypothesenfrei ermittelt werden. Die Anmelderin hat erkannt, dass insbesondere chronischen Erkrankungen grundsätzlich zu Veränderungen an der Zellmembran führen. Daher kann beispielsweise durch einen Vergleich der KBMs der Zellmembran und der KBMs der Golgi-Vesikel und/oder des Golgi-Apparats festgestellt werden, ob eine pathologische Abweichung der KBMs an der Zellmembran auf einer Verteilungs- und/oder Prozessierungsstörung in der Zellmembran beruhen oder ob bereits fehlerhaft verteilte und/oder prozessierte Proteine vom Golgi-Apparat zur Zellmembran geschickt werden. Im ersteren Fall wäre die pathologische Störung bzw. der einer Erkrankung zugrunde liegende Mechanismus in der Zellmembran selbst zu suchen, im zweiten Fall wäre die Ursache für die pathologische Abweichung im Golgi-Apparat oder in einem bezüglich des biologischen Informationsflusses der Zelle weiter stromauf liegenden Zellelement bzw. Zellkompartiment zu suchen.

Die Zellelemente werden ausgewählt aus der Gruppe DNA, RNA, Transkriptionsfaktor, Nukleolus, Nukleus, Endoplasmatisches Reticulum, CIS Golgi Vesikel, Golgi-Apparat, Vesikel, Trans-Golgi-Netzwerk, Zellmembran, Cytoskelett, Mitochondrien, Lysosom, Peroxisomen, Vesikel, Ribosomen, Mikrotubuli und Zentriolen. Durch Ermitteln der KBMs in wenigstens zwei dieser Zellelemente bzw. Zellkompartimente können grundsätzlich alle pathologischen Abweichungen der Zellprobe gegenüber einer nicht pathologisch veränderten bzw. gesunden Zellprobe ermittelt werden.

Anhand des Vergleichs der KBMs wird abgeleitet, ob zumindest eines der betrachteten Zellelemente pathologisch verändert ist. Beispielsweise anhand eines Vergleichs zwischen den KBMs der untersuchten Zellprobe und KBMs einer bekanntermaßen gesunden und der untersuchten Zellprobe entsprechenden Zellprobe kann ermittelt werden, ob die untersuchte Zellprobe pathologische Veränderungen aufweist.

In einer vorteilhaften Ausgestaltung der Erfindung wird eine Ligandenbibliothek verwendet, die wenigstens einen Zellelement-spezifischen Liganden und/oder wenigstens einen Liganden aus der Gruppe unspezifische Liganden, spezifische Liganden, markierte Liganden, monoklonale Antikörper, polyklonale Antikörper, rekombinante Antikörper, bispezifische Antikörper, Lectine, Nukleinsäuren, Nukleinsäuresequenzen, Peptide, Aptamere und Toxine umfasst. Hierdurch kann die Ligandenbibliothek optimal an das jeweils zu untersuchende Zellelement bzw. an die jeweils zu untersuchende Zellprobe angepasst werden. Unter einem Zellelement-spezifischen Liganden sind insbesondere Liganden zu verstehen, die spezifisch an einen im Zellelement vorhandenen und/oder an einen für das Zellelement charakteristischen Bestandteil binden.

Weitere Vorteile ergeben sich, indem eine Information bereitgestellt und/oder ermittelt wird, ob die Zellprobe pathologisch verändert ist, und/oder wenn anhand des Vergleichs der KBMs eine Zielstruktur für die Entwicklung eines Medikaments zur Behandlung eines Patienten, der unter einer durch eine pathologische Veränderung der betrachteten Zellprobe charakterisierten Erkrankung leidet, ermittelt wird. Es kann mit anderen Worten vorgesehen sein, dass von vornherein bekannt ist, ob die zu untersuchende Zellprobe pathologisch verändert ist bzw. von einem erkrankten Patienten stammt. Falls es sich bei der untersuchten Zellprobe um eine pathologisch veränderte Zellprobe handelt, wird anhand der KBMs der einzelnen Zellelemente der Mechanismus der pathologischen Veränderung ermittelt. Aufgrund der Kenntnis des Mechanismus wird dann eine diesem Mechanismus zugrunde liegende Zielstruktur ermittelt, die dann beispielsweise medikamentös zur Behandlung der entsprechenden Erkrankung beispielsweise mit Hilfe von Antikörpern gezielt angegriffen werden kann. Hiermit können chronische und/oder akute Erkrankungen unmittelbar am Ort ihrer zellulären Ursache gezielt und nebenwirkungsarm behandelt werden.

In einer weiteren vorteilhaften Ausgestaltung der Erfindung ist vorgesehen, dass in wenigstens einem der Zellelemente erneut KBMs mit Hilfe einer um wenigstens einen weiteren Liganden erweiterten Ligandenbibliothek ermittelt werden, wenn das wenigstens eine Zellelement als pathologisch verändert eingestuft wurde. Dies erleichtert einerseits aufgrund der verbreiterten Datenbasis die Entschlüsselung des grundlegenden Mechanismus einer pathologischen Veränderung, andererseits wird hierdurch vorteilhaft verhindert, dass gesunde bzw. nicht pathologisch veränderte Zellproben unnötiger Weise mit einer großen Zahl an Liganden untersucht wird, wodurch sich entsprechende Zeit- und Kostenvorteile ergeben.

In einer weiteren vorteilhaften Ausgestaltung der Erfindung ist vorgesehen, dass wenigstens ein weiteres KBM mit Hilfe einer wenigstens zwei Liganden umfassenden Ligandenbibliothek in wenigstens einem weiteren Zellelement wenigstens einer Zelle der Zellprobe ermittelt und mit wenigsten einem der bereits ermittelten KBMs verglichen wird, wenn ein Unterschied der bereits miteinander verglichenen KBMs eine vorbestimmte Unterscheidungsschwelle nicht überschreitet. Mit anderen Worten ist es erfindungsgemäß vorgesehen, dass in wenigstens einem weiteren Zellelement KBMs ermittelt und mit den bereits ermittelten KBMs verglichen werden, wenn die zuvor bestimmten KBMs einander zumindest im Wesentlichen entsprechen, so dass davon ausgegangen werden kann, dass die bereits untersuchten Zellelemente nicht pathologisch verändert oder zumindest nicht die zugrunde liegende Ursache für eine pathologische Veränderung sind.

In einer weiteren vorteilhaften Ausgestaltung der Erfindung ist vorgesehen, dass die KBMs in einer einzigen Zelle der Zellprobe und/oder in einem einzigen Gewebeschnitt und/oder in mehreren Zellen derselben Zellprobe und/oder in mehreren Zellen unterschiedlicher Zellproben ermittelt werden und/oder bei welchem wenigstens eines der KBMs mit Hilfe eines Fluoreszenzverfahrens, insbesondere eines multidimensionalen Fluoreszenzmikroskopieverfahrens, und/oder mit Hilfe eines MELK-Roboters und/oder mit Hilfe eines Toponome-Imaging-Systems ermittelt wird. Das beste Grundprinzip zur Bestimmung von ortsaufgelösten kombinatorischen Bindungsmustern (KBMs) sind fluoreszenzbasierte Verfahren, insbesondere Fluoreszenzmikroskopie. Um die hochdimensionale kombinatorische molekulare Organisation des Toponoms in ein und derselben subzellulären Struktur (Zellelement) zu erfassen, müssen mindestens zwei oder mehr Proteine und/oder andere Biomoleküle gleichzeitig in einer Zelle oder einem Gewebe kokartiert (co-mapped) werden. Wenn eine größere Menge von Liganden genutzt werden soll, insbesondere wenn mehr Liganden als verfügbare Wellenlängen innerhalb des Spektrums sichtbaren Lichts (zwischen etwa 300 und 700 nm) genutzt werden müssen, ist eine dimensional unbegrenzte molekulare Abbildungstechnik ratsam. Diese Theorie basiert auf großen Sondenbibliotheken (z.B. 20, 50, 100, 1,000 oder mehr Liganden), in denen jede Sonde oder jeder Ligand, die/der in situ spezifisch an eine bestimmte Gruppe oder ein bestimmtes Biomolekül bindet, mit ein und demselben Farbstoff gekoppelt wird, beispielsweise mit Fluoresceinisothiocyanat (FITC). Zusätzlich oder alternativ sind auch mehr als ein Farbstoff pro Ligand und/oder unterschiedliche Farbstoffe pro Ligand möglich. Das Prinzip sieht dann vor, dass ein Roboter (z.B. MELK/MELC-Roboter) automatisierte und vorprogrammierte wiederkehrende Zyklen der Schritte (i) Inkubation mit Farbstoffsonden auf dem Objekttisch eines Epifluoreszenz-Mikroskops; (ii) Abbilden des sich ergebenden Signals; und (iii) Inaktivierung dieses Signals mittels weichem Fluoreszenzbleichen und/oder Inaktivierung dieses Signals unter Verwendung eines entsprechenden chemischen Wirkstoffes durchläuft. Diese auf den sogenannten "Venedig"- und "Sonnenlicht"-Hypothesen basierende Theorie eines dimensions- und parameterunlimitierten molekularen Abbildens wurde experimentell verifiziert, nachdem mehrere Generationen entsprechender Roboter (Multi-Epitope Ligand Cartography, MELC, und Toponome Imaging Systems, TIS) entwickelt wurden, die alle als Imaging Cycler bezeichnet werden.

In einer weiteren vorteilhaften Ausgestaltung der Erfindung ist vorgesehen, dass wenigstens eines der ermittelten KBMs als Toponomdatensatz in einer Datenbank einer Recheneinrichtung gespeichert wird und/oder bei welchem zum Vergleichen der KBMs Toponomdatensätze der entsprechenden KBMs mit Hilfe der Recheneinrichtung bereitgestellt und miteinander verglichen werden. Die Recheneinrichtung kann grundsätzlich mehrere Rechen- und/oder Speichereinheiten umfassen. Es kann vorgesehen sein, dass die Recheneinrichtungen über ein drahtloses und/oder kabelgebundenes Netzwerk miteinander verbunden sind. Weiterhin können die Toponomdatensätze teilweise oder vollständig delokal, verteilt bzw. cloud-basiert gespeichert sein.

Zum Durchführen eines Verfahrens nach einem der vorhergehenden Ausführungsbeispiele eignet sich eine Vorrichtung, die wenigstens eine Einrichtung zum Ermitteln von ortsaufgelösten kombinatorischen Bindungsmustern (KBM) mit Hilfe einer wenigstens zwei Liganden umfassenden Ligandenbibliothek in wenigstens zwei Zellelementen wenigstens einer Zelle einer Zellprobe und wenigstens eine Einrichtung zum Vergleichen der ermittelten KBMs umfasst.

Es kann vorgesehen sein, dass die Einrichtung zum Ermitteln der ortsaufgelösten kombinatorischen Bindungsmuster (KBM) wenigstens eine Messeinrichtung umfasst, mittels welcher das Bindungsverhalten wenigstens eines Liganden aus der Ligandenbibliothek in der Zellprobe ortsaufgelöst ermittelbar ist. Die wenigstens Messeinrichtung kann dabei beispielsweise als automatisiertes multidimensionales Fluoreszenzmikroskop (Toponome Imaging System, TIS) ausgebildet sein, welches das Bindungsverhalten jedes Liganden der Ligandenbibliothek in der untersuchten Probe, beispielsweise in einer einzelnen Zelle, in mehreren Zellen, in einem Gewebeschnitt etc., durch repetitives Beaufschlagen der Probe mit den einzelnen Liganden und Ermittlung des Bindungsverhaltens der einzelnen Liganden in situ bestimmen kann. Die Messeinrichtung kann weiterhin ausgebildet sein, die Zellprobe mehrfach und/oder in randomisierter Reihenfolge und/oder in einer vorbestimmten Reihenfolge mit den Liganden der Ligandenbibliothek zu beaufschlagen, um Fehlmessungen durch Kreuzreaktionen zwischen Liganden, Maskierung von Bindungsstellen durch andere Liganden usw. besonders zuverlässig auszuschließen.

Es kann vorgesehen sein, dass die Einrichtung zum Ermitteln von ortsaufgelösten KBMs wenigstens zwei Messeinrichtungen umfasst, die über ein Netzwerk zum Austauschen von Daten miteinander verbunden sind. Hierdurch ist eine besonders schnelle und zuverlässige Ermittlung der KBMs und eine entsprechend schnelle und zuverlässige Ermittlung von krankheitsspezifischen kombinatorischen Biomarkern (TIS-Codes) durch Vergleich der ermittelten KBMs mit KBMs einer gesunden Zellprobe ermöglicht. Weiterhin kann vorgesehen sein, dass die wenigstens zwei Messeinrichtungen mit gleichen und/oder unterschiedlichen Ligandenbibliotheken bestückt sind bzw. werden. Im Fall gleicher Ligandenbibliotheken können die wenigstens zwei Messeinrichtungen gleichzeitig ortsaufgelöste kombinatorische Bindungsmuster (KBM) in einem bestimmten Zellelement einer einheitlichen Zellprobe oder in einem bestimmten Zellelement unterschiedlicher Zellproben ermitteln. Die ermittelten KBMs können dann vergleichen werden, um Aussagen über Unterschiede zwischen den jeweils vermessenen Zellproben, insbesondere im Hinblick auf pathologische Veränderungen und Abweichungen treffen zu können. Alternativ oder zusätzlich können die wenigstens zwei Messeinrichtungen mit unterschiedlichen Ligandenbibliotheken bestückt werden, so dass eine der Messeinrichtungen die KBMs in einem ersten Zellelement bzw. Zellkompartiment ermittelt, während die andere der Messeinrichtungen die KBMs in einem bezüglich der Reihenfolge des biologischen Informationsflusses hierarchisch vor- und/oder nachgeschalteten Zellelement bzw. Zellkompartiment ermittelt. Beispielsweise kann eine Messeinrichtung mit einer ersten Ligandenbibliothek bestückt werden, die spezifisch für die Untersuchung von Zellmembranen ausgelegt ist, während wenigstens eine andere Messeinrichtung mit einer zweiten Ligandenbibliothek bestückt wird, welche spezifisch für die Untersuchung des Golgi-Apparats ausgelegt ist. Grundsätzlich können die einzelnen Ligandenbibliotheken zur Detektion von Zellelementen aus der Gruppe DNA, RNA, Transkriptionsfaktor, Nukleolus, Nucleus, Endoplasmatisches Reticulum, CIS Golgi Vesikel, Vesikel, Trans-Golgi-Netzwerk, Zellmembran, Cytoskelett, Mitochondrien, Lysosom, Peroxisomen, Vesikel, Ribosomen, Mikrotubuli, Zentriolen oder sonstiger biologischer Elemente ausgelegt werden. Ebenso kann vorgesehen sein, dass jede Messeinrichtungen mit jeweils einer krankheitsspezifischen Ligandenbibliothek bestückt wird, so dass gleichzeitig oder nacheinander auf das Vorliegen unterschiedlicher pathologischer Veränderungen geprüft werden kann. Mit Hilfe der Netzwerkverbindung können die wenigstens zwei Messeinrichtungen Daten gesteuert bzw. geregelt und aufeinander abgestimmt werden. Auf diese Weise können beispielsweise TIS-Toponom-Cluster gebildet werden, die als eine Art virtuelle Fabrik bzw. als räumlich verteilte und miteinander vernetzte Analysestationen betrieben und koordiniert werden können.

Es kann vorgesehen sein, dass die wenigstens eine Messeinrichtung mit einer Datenbank gekoppelt und ausgebildet ist, Bindungsdaten, die das ortsaufgelöste Bindungsverhalten des wenigstens einen Liganden in der Zellprobe charakterisieren, in der Datenbank zu speichern. Hierdurch können die von der Messeinrichtung ermittelten Bindungsdaten, das heißt beispielsweise die gemessenen 2D- und/oder 3D-Toponom-Datensätze, effizient, widerspruchsfrei und dauerhaft zentral gespeichert werden. Benötigte Teilmengen der Datenbank können zudem in unterschiedlichen, bedarfsgerechten Formen für Benutzer,
Anwendungsprogramme und zur Steuerung und/oder Regelung weiterer Messeinrichtungen bereitgestellt werden. Beispielsweise kann über ein Netzwerk auf die Datenbank zugegriffen werden, um ein als Similarity Mapping (SIM, Ähnlichkeitsabbildung) bezeichnetes Verfahren auf die gespeicherten Daten anzuwenden und auf einem Anzeigegerät auszugeben. Dieses TIS-SIM-Verfahren kann grundsätzlich in Echtzeit durchgeführt werden, was es einem Histologen erlaubt, die vermessene(n) Zellprobe(n) wie bei einem elektronischen Mikroskop parallel zur normalen Hellfeldmikroskopie in der routinemäßigen histologischen Diagnostik zu beurteilen. Hierbei können beispielsweise Datenpunkte optisch hervorgehoben oder anderweitig kenntlich gemacht werden, die als pathologisch charakterisierte Protein- bzw. Ligandenbindungsprofile kennzeichnen.

Es kann vorgesehen sein, dass die Datenbank mit einer Recheneinrichtung gekoppelt ist, wobei die Recheneinrichtung ausgebildet ist, die ortsaufgelösten kombinatorischen Bindungsmustern (KBM) anhand der Bindungsdaten zu ermitteln und/oder die ermittelten KBMs zu vergleichen und/oder die Art und/oder Anzahl der Liganden in der Ligandenbibliothek zu verändern und/oder die wenigstens eine Messeinrichtung zu steuern und/oder zu regeln. Mit anderen Worten ist eine Recheneinrichtung vorgesehen, welche wahlweise die jeweiligen KBMs anhand der von den Messeinrichtungen gemessenen Bindungsmuster ermittelt und/oder die ermittelten KBMs vergleicht und/oder die wenigstens eine Messeinrichtung insbesondere in Abhängigkeit der ermittelten KBMs steuert und/oder regelt. Die Steuerung und/oder Regelung kann dabei grundsätzlich die Anpassung der Ligandenbibliothek derselben Messeinrichtung und/oder einer anderen Messeinrichtung und/oder mehrerer Messeinrichtungen umfassen. Ebenso kann die Steuerung und/oder Regelung den Start und/oder den Abbruch und/oder die Änderung eines Ablaufprogramms wenigstens einer Messeinrichtung umfassen.

Weitere Merkmale der Erfindung ergeben sich aus den Ansprüchen, den Ausführungsbeispielen sowie anhand der Zeichnungen. Die vorstehend in der Beschreibung genannten Merkmale und Merkmalskombinationen sowie die nachfolgend in den Ausführungsbeispielen genannten Merkmale und Merkmalskombinationen sind nicht
nur in der jeweils angegebenen Kombination, sondern auch in anderen Kombinationen verwendbar, ohne den Rahmen der Erfindung zu verlassen. Dabei zeigt:
- Fig. 1: eine Prinzipdarstellung eines Untersuchungsverfahrens, bei welchem ortsaufgelöste kombinatorische Bindungsmuster (KBM) in mehreren Zellelementen einer Zellprobe entgegen der Reihenfolge des biologischen Informationsflusses ermittelt werden (Reverse Imaging);
- Fig. 2: eine Prinzipdarstellung eines Untersuchungsverfahrens, bei welchem in einer Zellprobe in situ zelluläre Mechanismen mit Hilfe von hochdimensionalen molekularen Filtern (high-dimensional molecular filters, HDM) dekodiert werden;
- Fig. 3: ein schematischer Vergleich zwischen den Toponomen einer gesunden und einer pathologisch veränderten Zellprobe;
- Fig. 4: eine schematische Darstellung der topologischen und funktionellen Hierarchien mehrerer Proteine in einem Toponom;
- Fig. 5: eine Prinzipdarstellung, in welcher die funktionelle Hierarchie vom KBMs dargestellt ist;
- Fig. 6 bis Fig. 10: eine Prinzipdarstellung eines Untersuchungsverfahrens, bei welchem KBMs in einer Zellprobe ermittelt und räumlich dargestellt werden;
- Fig. 11 bis Fig. 16: verschiedene Abbildungen einer Basallamina-Zellprobe mit einem stöchiometrisch kontrollierten Supermolekül;
- Fig. 17: eine Prinzipdarstellung eines TIS-Clusters; und
- Fig. 18: ein beispielhaftes Ablaufdiagramm eines Reverse-Imaging-Verfahrens.

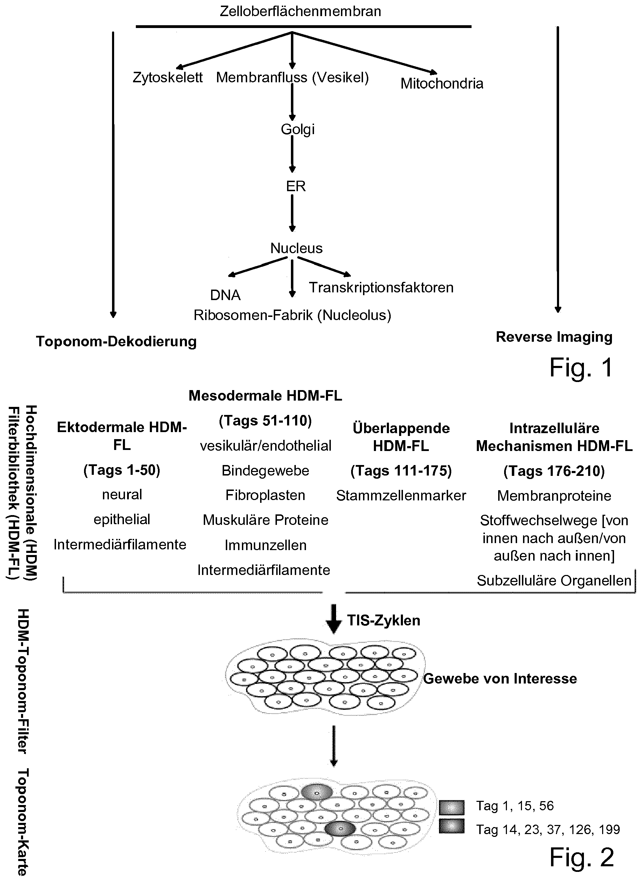

Fig. 1 zeigt eine Prinzipdarstellung eines Untersuchungsverfahrens, bei welchem ortsaufgelöste kombinatorische Bindungsmuster (KBM) in mehreren Zellelementen einer Zellprobe entgegen der Reihenfolge des biologischen Informationsflusses ermittelt werden (Toponome decoding by reverse imaging). Alle Schritte können grundsätzlich in ein- und derselben Zelle oder in ein- und demselben Gewebeschnitt durchgeführt werden. Fakultativ können auch Serienschnitte oder Zellen des gleichen Typs verwendet werden.

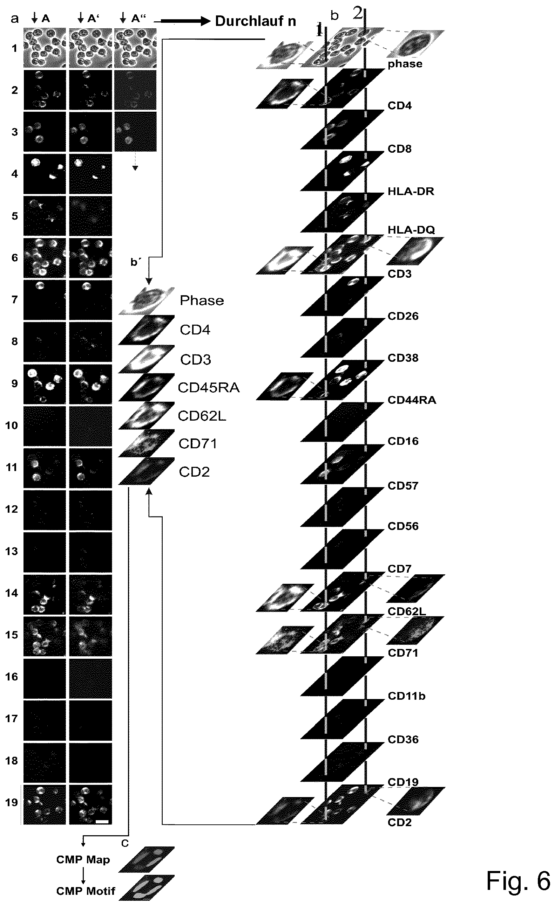

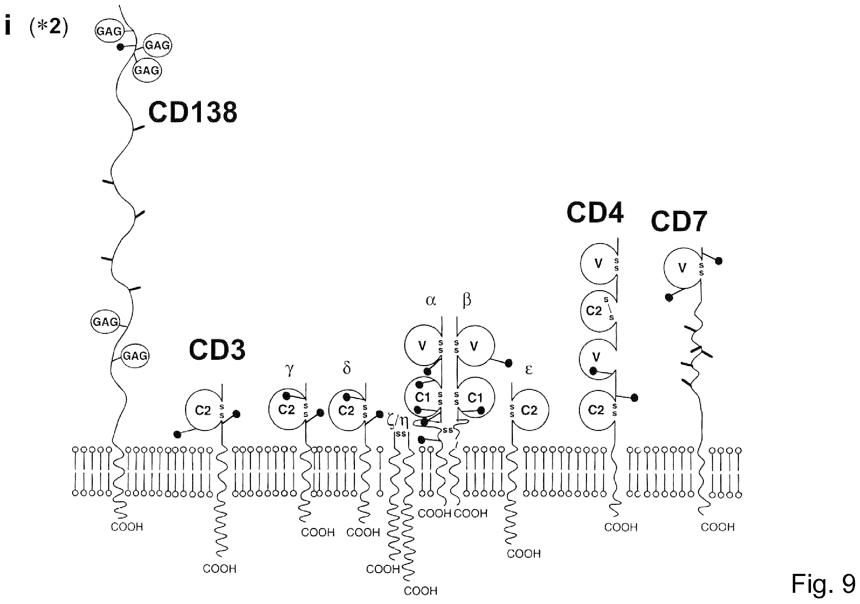

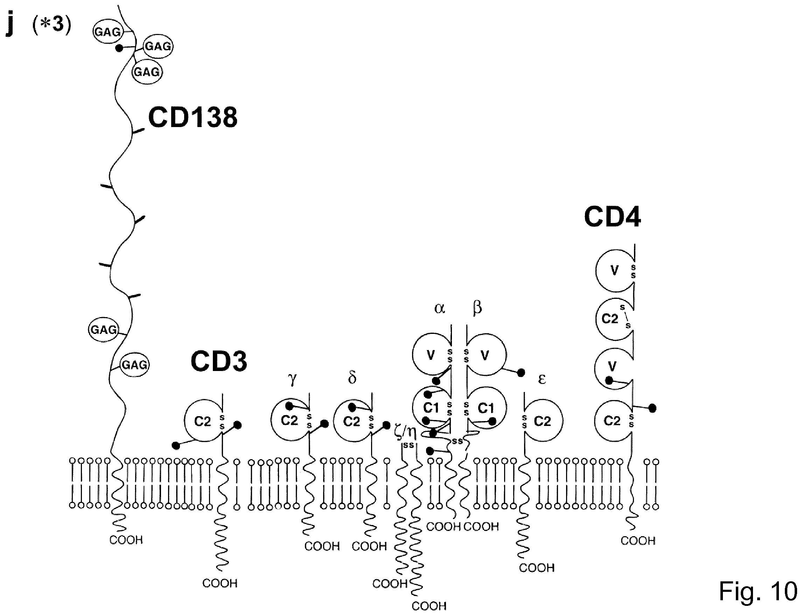

In Schritt 1 wird wenigstens eine Zelloberflächenmembran mit Hilfe der ortsaufgelösten Kokartierung (s. Fig. 6 bis Fig. 10) einer Antikörper-/Liganden-Bibliothek (Bibliothek 1) gegen mindestens 2 bis 15 verschiedene Zelloberflächenproteine untersucht. In Schritt 2 wird das Resultat (=Protein-/Liganden-Cluster-Information) in Form eines oder mehrerer 2D oder 3D Toponom-Datensätze elektronisch gespeichert. In Schritt 3 wird die Bibliothek aus Schritt 1 um weitere Antikörper/Liganden gegen Vesikelständige Proteine (Bibliothek 2) erweitert, die im sogenannten Trans-Golgi Netzwerk (TGN, exozytotischer Transport) spezifisch lokalisiert sind (Bibliothek 1 + Bibliothek 2 = Bibliothek 3). In Schritt 4 werden die Proteine kokartiert, die aus der erweiterten Bibliothek 3 innerhalb derselben Zelle auf diese Weise zu erkennen sind, und erneut als 2D oder 3D Toponom Datensätze gespeichert.

In Schritt 5 werden die Protein/Biomolekül-Cluster der Zelloberfläche (aus Bibliothek 1) mit den Protein Clustern der Vesikel (aus Bibliothek 3 - Bibliothek 2) verglichen und das Ergebnis gespeichert. In Schritt 6 wird geprüft, ob die Protein/Biomolekül - Cluster der Zelloberfläche mit denen innerhalb der Vesikel identisch oder zumindest sehr ähnlich sind. Dies erlaubt eine Schlussfolgerung darüber, ob die in der Zelloberfläche bzw. Zellmembran ermittelten Protein/Biomolekül-Cluster nicht innerhalb der Oberflächenmembran, sondern innerhalb der Golgi-TGN Mechanismen der Protein Sortierung im Exocytose Weg der Zelle entstanden und auf diese Weise in die Zelloberfläche transportiert worden sind.

Ergibt die Prüfung in Schritt 6, dass die Protein/Biomolekül-Cluster nicht bzw. nicht ausreichend ähnlich sind, so wird in Schritt 7 die Schlussfolgerung abgeleitet, dass die Protein-/Biomolekül Cluster der Zelloberflächenmembran innerhalb dieser Membran und nicht innerhalb des Golgi-mediierten Membran-Vesikel Transportwegs entstanden sind.

Handelt es sich bei dem untersuchten Material um pathologisches Zell- oder Gewebematerial bzw. um Zell- oder Gewebematerial einer Krankheit bzw. eines erkrankten Patienten, so ist im Falle von Schritt 6 gemäß einem Schritt 8 zu schließen, dass die Krankheitsursache in einer abnormen Protein-Ko-Sortierung im Gogi Apparat liegt.

Handelt es sich bei dem untersuchten Material um Zell- oder Gewebematerial einer Krankheit, so ist im Falle von Schritt 7 gemäß einem Schritt 9 zu schließen, dass die Krankheitsursache bzw. der krankhafte Mechanismus in einer abnormen Protein-Ko-Sortierung innerhalb der Zelloberflächenmembran liegt.

Im Falle von Schritt 7 wird die Ligandenbibliothek in Schritt 10 um eine entsprechende Anzahl an Antikörper/Liganden (z. B. mehrere tausend) erweitert, die möglichst alle oder zumindest einen überwiegenden Teil aller Oberflächenproteine erkennen und binden.

Im Falle von Schritt 8 wird die Bibliothek in Schritt 11 um eine entsprechende Anzahl an Antikörpern/Liganden (mehrere tausend) erweitert, die möglichst alle oder zumindest einen überwiegenden Teil aller Golgi- bzw. Vesikel-residenten Proteine erkennen und binden.

In beiden Fällen (Schritt 10 und 11) zeigt sich in einem weiteren Schritt 12 (ggf. im Vergleich mit gesunden Zellen/Gewebeschnitten) die molekulare Ursache einer pathologischen Störung in Form eines ganz spezifischen Protein-/Biomolekül-Clusters, das nur bei der konkreten Krankheit innerhalb der analysierten Zellkompartimente (Oberflächenmembran oder Golgi-TGN Apparat usw.) vorkommt.

Um den Weg des "Reverse Imaging" weiter fortzusetzen, kann in einem weiteren Schritt 13 die Ligandenbibliothek fakultativ um weitere Teilbibliotheken bzw. Liganden erweitert werden, welche eine oder mehrere der folgenden Funktionen/Strukuren/Moleküle erfassen:
(i) den Vesikel Transport vom Endoplasmatischen Retikulum (ER) zum Golgi Apparat (CIS Golgi Vesikel);
(ii) ER residente Proteine;
(iii) Proteine im Zellkern (Transkriptionsfaktoren, welche die Genexpression regulieren);
(iv) molekulare Komponenten, die im Nucleolus an der Biogenese der Ribosomen beteiligt sind);
(vi) Nucleinsäuresequenzen von DNA und RNA mit oder ohne ko-kartierte Transkriptionsfaktoren, um krankhafte Veränderungen im Genregulations- und Transkriptionsapparat festzustellen.

In einem grundsätzlich fakultativen Schritt 14 kann nach Schritt 13 und/oder vor Schritt 1 und/oder zu jedem beliebigen Zeitpunkt zwischen Schritt 1 und 13 die Zelloberflächen-Bibliothek 1 durch eine Teilbibliothek C erweitert werden, welche beispielsweise Proteine des Cytoskeletts erkennt (Actin, Tubulin, Intermedärfilamente etc.), um festzustellen, mit welchem zelltypspezifischen Cytoskelett die Protein/Biomolekül-Cluster eines der Kompartimente Zelloberfläche, TGN Vesikel, Golgi, ER, Nucleus, DNA, Transkriptionsfaktoren und/oder Nucleolus direkt assoziiert sind. Diese Kokartierung gibt unmittelbar Auskunft über die Keimblattherkunft des untersuchten Gewebes bzw. Zelltyps und auch darüber, ob die untersuchte Zelle gegebenenfalls neuartige, nicht keimblattkonforme Kriterien aufweist, also beispielsweise weder rein ektodermalen, mesodermalen oder entodermalen Urspungs und gegebenenfalls eine Keimblattchimäre ist. Derartige Chimären wären z.B. dann gegeben, wenn spezifische Zelloberflächenprotein-Cluster aus einem Lymphozyten (mesodemale Herkunft) zusammen mit solchen einer Epithelzelle und/oder zusammen mit dem Intermediärfilament Cytokeratin (ectodermaler Herkunft) vorkommen, was auf ein neuartiges hochpathogenes (ggf. kanzerogenes) Keimblatt bzw. einen ektodermal-mesodermalen Transdifferenzierungsvorgang als kausalen Krankheitsmechanismus schließen lässt.

Die Ermittlung und gegebenenfalls Visualisierung solcher Zusammenhänge sind fundamental für eine gezielte Therapie, da als krankheitsspezifisch ermittelte Protein-/Biomolekül-Cluster (an der Zellmembran und/oder andere intra- und/oder extrazelluläre Protein-/Biomolekül-Cluster) spezifische molekulare Anweisungen und damit Zielstrukturen für die Generierung hoch selektiver Therapeutika liefern.

Alle Anweisungen unter Schritt 1 bis 14 schließen ein, dass jede Bibliothek bzw. Ligandenbibliothek vorzugsweise hoch affine Liganden enthält, die Biomoleküle binden, also beispielsweise Antikörper, Lectine, Nucleinsäuren, Nucleinsäuresequenzen, Peptide, Aptamere, Toxine, usw. Fakultativ kann die Ligandenbibliothek an jeder Stelle der Schritte 1 bis 14 auch um eine Bibliothek M erweitert werden, die Biomoleküle erkennt, welche für Mitochondrien spezifisch sind. Die Bibliothek M kann isoliert oder in jeder Kombination mit jeder der anderen genannten Teilbibliotheken verwendet werden. Fakultativ kann auch jede Ligandenbibliothek einzeln und ohne die Anweisungen von spezifischen Abfolgen des Reverse Imaging-Verfahrens verwendet werden.

Der Vorteil des Verfahrens (Toponome decoding through Reverse Imaging) besteht darin, dass Krankheitsmechanismen am Zielort der Krankheit im menschlichen oder tierischen Gewebe unmittelbar entschlüsselt werden können, ohne auf indirekte Modelle (Mausmodelle, Zellmodelle) zurückgreifen zu müssen, die den erwiesenen Nachteil sehr geringer Prädiktivität für den Menschen mit sich bringen (Literatur: Hutchinson L, Kirck R. High drug attrition rates - where are we going wrong? Nat Rev Clin Oncol 2011; 8, 189 - 190). Darüber hinaus besteht die Möglichkeit der Gesamtentschlüsselung des Toponoms für bis zu 20000 oder mehr verschiedene menschliche/tierische Proteine/Biomoleküle durch die Verfügbarkeit von (humanen) rekombinanten Antikörpern. Dieses "Toponome decoding" kann grundsätzlich mit Genome Sequencing an ein und demselben Gewebe durchgeführt werden (sog. TIS Toponome Cluster, die ggf. als virtuelle Fabrik bzw. als räumlich verteilte und miteinander vernetzte Analysestationen vorgesehen sein können).

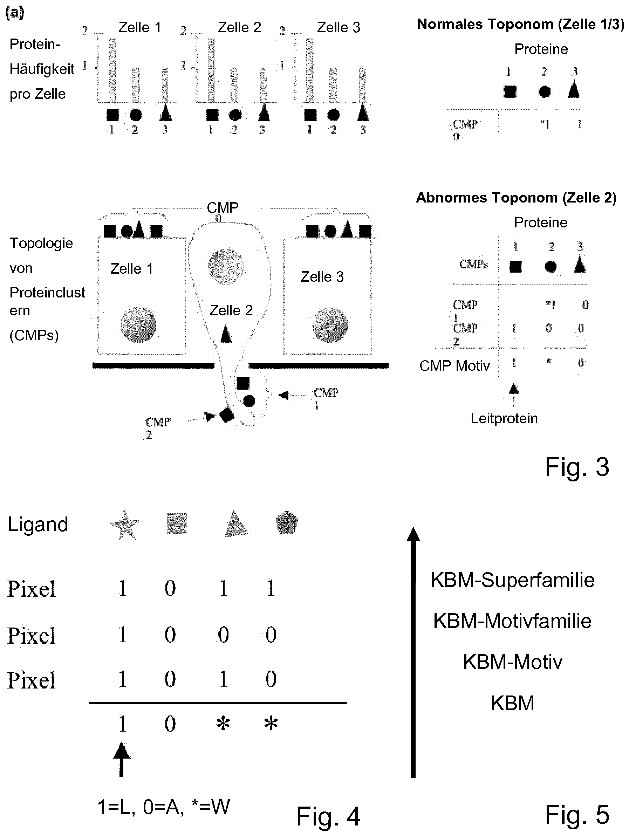

Fig. 2 zeigt eine Prinzipdarstellung eines Untersuchungsverfahrens, bei welchem in einer Zellprobe in situ zelluläre Mechanismen mit Hilfe von hochdimensionalen molekularen Filtern (high-dimensional molecular filters, HDM) dekodiert werden (Decoding cellular mechanisms *in situ* by using high-dimensional molecular filters). Fig. 2 wird im Folgenden in Zusammenschau mit Fig. 3 erläutert werden, welche einen schematischen Vergleich zwischen den Toponomen einer gesunden und einer pathologisch veränderten Zellprobe zeigt. Ein hochdimensionaler molekularer (HDM-) Filter ist eine große Sondenbibliothek (probe (tag) library), die beispielsweise von TIS angewandt wird, um auf visuelle und algorithmische Weise spezifische Toponommerkmale in einer Mischung morphologisch intakter Zellen/Zellstrukturen zu detektieren und zu isolieren, wobei die Zellen/Zellstrukturen in einem hochorganisierten Gewebe oder in einer Zellprobe vorhandenen sind. Beispielsweise können Epithelzellen, die bei einer normalen Einfärbung homogene Strukturen aufweisen, ausgesprochen einzigartige Merkmale aufweisen, wenn sie unter Verwendung eines 100-dimensionalen HDM-Filters visualisiert werden. Ohne diesen HDM-Filter können die genannten funktionellen Zelltypen nicht detektiert werden. Gewöhnlich werden HDM-Filter als Strategie zur gleichzeitigen Identifizierung verschiedener Funktionsarten von (i) unterschiedlichen Zelltypen, (ii) Zelllinien mesodermalen und ectodermalen Ursprungs zusammen mit (iii) deren einzigartigem Satz an Membranproteinen, Proteinen des Sekretionsweges und einem Satz an Proteinen, die für subzelluläre Organellen charakteristisch sind, eingesetzt. Hdm-Filter können eingesetzt werden, wenn beispielsweise wenig oder nichts bekannt ist über den zelllären Ursprung und/oder die Pathogenese einer (chronischen) Krankheit in Zielstrukturen des Gewebes und wenn die Identifizierung von Schlüsselzellen und deren pathogenen Leitproteinen entscheidend sind für die Entwicklung von Medikamenten, die therapeutisch auf diese Zellen gerichtet sind.

Hdm-Filter sind wichtig bei der Identifizierung von Zellursprüngen von Krebs oder komplexen morphogenetischen Regenerationsmechanismen. Beispielsweise können HDM-Filter zur Identifizierung einer (prä)kanzerösen Epithelzelle, die bereit ist, über die Basalschicht zu wandern, verwendet werden. Gleichzeitig wird die einzigartige und pathogene Art der Umordnung der KBMs (CMPs) an der Spitze der Zelle, die die Zellmigration auslöst, von demselben HDM-Filter ermittelt. Wichtig ist, dass sobald der erste Satz CMPs von einem ersten breiten HDM-Filter ermittelt wurde, ein zweiter HDM-Filter angewendet werden kann, um die spezielle subzellulären Sekretionswege zu dekodieren, die diese Zelle im Gegensatz zu nicht-kanzerösen Epithelzellen in suprabasaler Umgebung benutzt. Daher wird eine Strategie einer nurgleichzeitigen oder sequenziellen Hdm-Filterung bei demselben Gewebe zu sicheren *in situ* Identifizierung von krebsspezifischen Sekretionswegen, Mechanismen und Zielstrukturen für Arzneimittelkandidaten führen. Gemäß dem im Schema von Fig. 3 und 4 dargestellten Leitproteinkonzept ist das hervorstechende Leitprotein für Arzneimittelkandidaten in dem CMP-Komplex an der Grenze der (prä)kanzerösen Epithelzelle, die Basallamina penetriert. Gewebe halten diesen HDM-Filterungsprozessen etwa sechs Monate lang stand, ohne ihre räumliche Toponomstruktur zu verändern.

Ein wichtiger Vorteil der HDM-Filterung besteht darin, dass tierische und vorklinische Modelle, die häufig nicht repräsentativ für chronische Krankheiten beim Menschen sind, vermieden werden können, und dass die Krankheitsmechanismen direkt an den krankheitsspezifischen Zielstellen im menschlichen Gewebe identifiziert werden können.

Fig. 4 zeigt eine schematische Darstellung der topologischen und funktionellen Hierarchien mehrerer Proteine in einem Toponom (grundlegende Struktur- und Funktionseinheiten des Toponoms). Wie jedes System setzt sich das Toponom aus Untereinheiten (Funktionseinheiten) unterschiedlicher Größenordnungen zusammen - KBMs, KBM-Motive und KBM-Superfamilien (Fig. 5). Sowohl die Proteinhierarchie als auch die in diesen Strukturen zu findenden Leitproteine/Biomoleküle sind der Schlüssel zu Vorhersagbarkeit und therapeutischen Effizienz, wie durch toponomische Studien aufgezeigt wird: Leitproteine (L), die durch Proteinkokartierung auf der Oberfläche von Tumorzellen ermittelt werden, können erstrangige Zielstruktur-Moleküle sein, weil sie sowohl die subzelluläre Topologie als auch die Funktion großer Zelloberflächenproteinnetzwerke steuern, wie durch die Entdeckung des "Aminopeptidase Polarisation Control Network" (APOCON) mit Hilfe der Toponomics aufgezeigt wurde:
wenn das ermittelte, mit der Zelloberfläche assoziierte Leitprotein (CD13) durch ein kleines Molekül blockiert oder inhibiert ist, dann zerfällt das entsprechende, entlang der Zelloberflächenmembran angeordnete Proteinnetzwerk vollständig, was zu einem Verlust der Funktion der Tumorzelle führt, vom sphärischen Zustand in den Erkundungszustand ("explore" Zustand) übergehen zu können, wodurch sie an der Metastasierung gehindert ist. Diese Beobachtung hat gezeigt, dass die Ermittlung von Leitproteinen in situ (und nicht durch umfangreiche ex vivo Expressionsprofile) ein wesentlicher erster Schritt auf dem Weg zur Entwicklung effizienter Therapien sein kann, indem der hypothesefreie Ansatz der Toponomdekodierung angewendet wird. Grundsätzlich besitzt Toponomics die Fähigkeit, relevante Leitproteine bei einer großen Bandbreite menschlicher Proteine in jeglicher Art von Gewebe zu ermitteln, indem verschiedene schwellenwertbasierte und nicht-schwellenwertbasierte Verfahren zur Erfassung des hochdimensionalen kombinatorischen Molekularcodes von Proteinsystemen eingesetzt werden, und zwar gleichzeitig in Millionen von subzellulären Datenpunkten. Beispielsweise war es möglich, mehr als 2000 unterschiedliche Proteincluster in einem einzigen Prostatakrebs-Gewebeschnitt und mehr als 5000 verschiedene Proteincluster in einem Darmkrebs-Gewebeschnitt in situ zu ermitteln. Dies hat gezeigt, dass Krebsmechanismen häufig auf eine subzelluläre Proteinumordnung beschränkt sind und nicht auf Hoch- oder Runterregulierungen ihrer Häufigkeit. Dies kann aber durch ex vivo Expressionsprofile nicht nachgewiesen werden.

Fig. 4 und Fig. 5 zeigen schematische Darstellungen der topologischen und funktionalen Hierarchien von Proteinen innerhalb des Toponoms. (a) In einem biologischen System, wie etwa einer Zelle oder einem Gewebe, können eine beliebige Anzahl unterschiedlicher Proteine (Symbole in der obersten Zeile) verschiedene kombinatorische Molekularphänotypen (KBM 1, KBM 2,...) an einem oder mehreren subzellulären Datenpunkten bilden. Sie können gemeinsame Merkmale haben und dadurch eine funktionale Gruppe bilden, die als KBM-(CMP)-Motiv bezeichnet wird, mit: L=Leitprotein(e)/Ligand(en) (allen KBMs eines KBM-Motivs gemeinsam); A, abwesende Protein(e)/Ligand(en) (abwesend in allen KBMs eines KBM-Motivs); W, Platzhalterproteine (Proteine/Liganden, die variabel mit den (L) und den (A) Proteinen eines Motivs assoziiert sind). (b) Funktionale Hierarchie der in KBM-Motive, KBM-Motivfamilien, KBM-Superfamilien usw. gruppierten KBMs, wobei diese KBMs mindestens ein gemeinsames Leitprotein/einen gemeinsamen Liganden aufweisen.

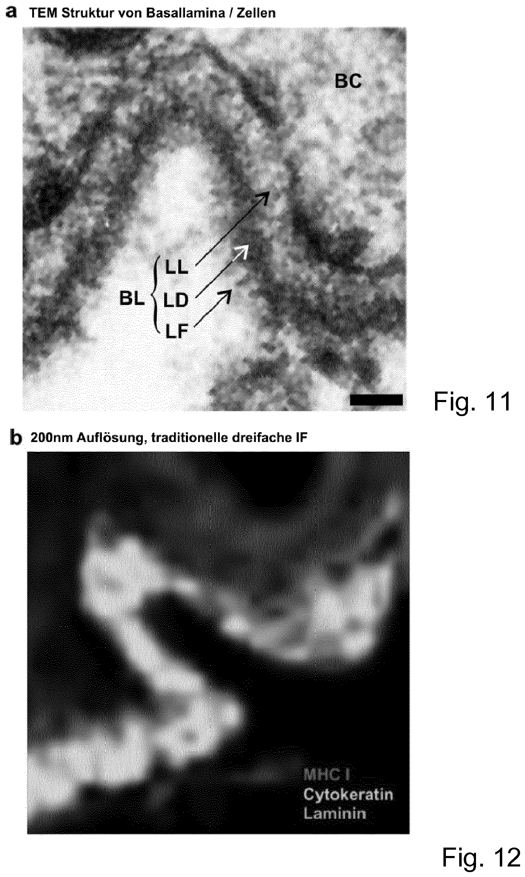

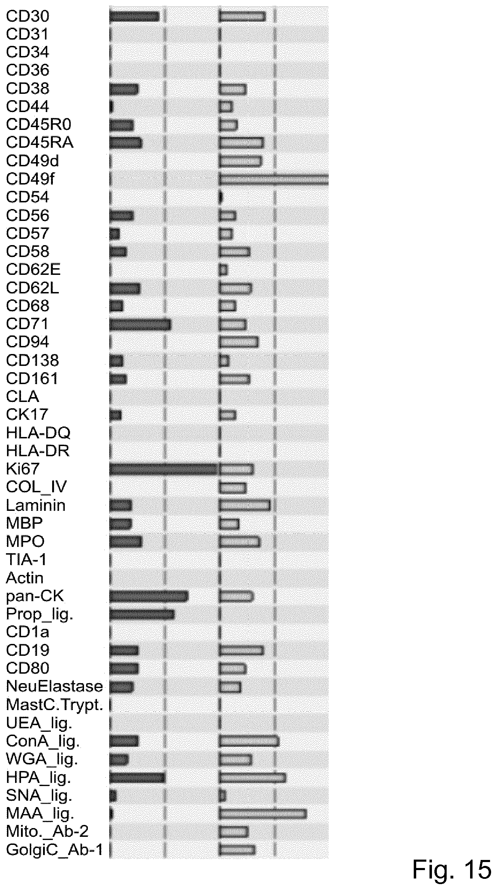

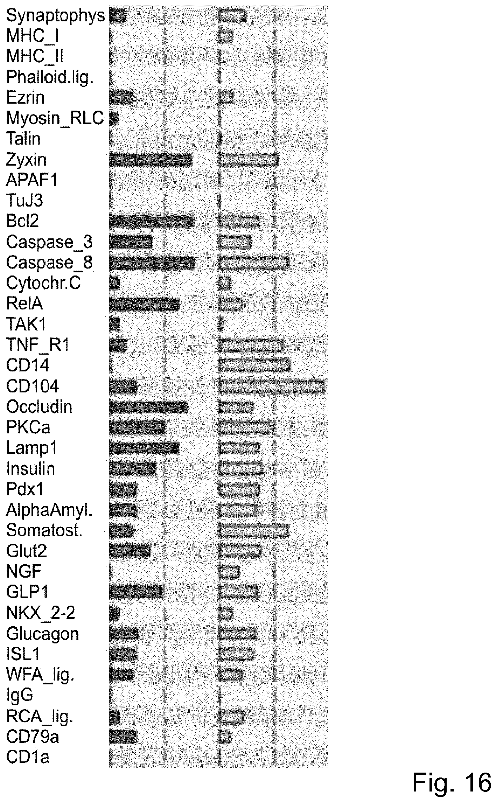

Der grundlegende Aspekt der Technologie zur Dekodierungsfähigkeit von Proteinnetzwerken und zur Ermittlung ihrer Selektivität und Spezifität in intakten Geweben ist ihre Unterscheidungskraft bezüglich kombinatorischer Molekülmuster (power of combinatorial molecular discrimination, PCMD) pro subzellulärem Datenpunkt. Wenn beispielsweise ein TIS-Messvorgang die Kokartierung von 100 unterschiedlichen Protein- und Kohlehydratgruppen umfasst, ist die sich ergebende PCMD 2¹⁰⁰, vorausgesetzt, dass jedes Fluoreszenzsignal einer jeden gegebenen funktionellen Gruppe aus diesen 100 unterschiedlichen Gruppen als anwesend oder abwesend im Verhältnis zu einem Schwellwert (automatisch bestimmt, oder bestimmt durch Fachleute: 1 Bit pro Protein) festgestellt wird. Wenn die Signale ohne jeglichen Schwellwert registriert werden, indem ein als Similarity Mapping (SIM, Ähnlichkeitsabbildung) bezeichnetes Verfahren angewendet wird, ist die sich ergebende PCMD 256¹⁰⁰ pro Datenpunkt (Fig. 11 bis Fig. 16). Dieses TIS-SIM-Verfahren wird gänzlich in Echtzeit durchgeführt, was es dem Histologen erlaubt, es wie ein elektronisches Mikroskop parallel zur normalen Hellfeldmikroskopie in der routinemäßigen histologischen Diagnostik zu verwenden, um Pixel durch Hervorhebung zu ermitteln, die die identischen Proteinprofile aufweisen, während der manuell gesteuerte Cursor sich über das Gewebe bewegt.

Fig. 11 bis Fig. 16 liefern ein Beispiel, das die hohe PCMD von 256¹⁰⁰ pro Datenpunkt verdeutlicht, wobei zwischen den drei Schichten der Basallamina der Haut mit einem Durchmesser von insgesamt 120 nm unterschieden wird, wie dies aus der Transmissionselektronenmikroskopie bekannt ist. Dieses Größenverhältnis ist relativ stabil für alle Basallaminae bei allen menschlichen Geweben, sowie für die drei ultrastrukturell unterscheidbaren Basallaminaschichen (BL): lamina lucida, lamina densa, und lamina fibroreticularis. Wie in Fig. 11 bis Fig. 16 gezeigt, kann TIS-SIM alle diese drei Schichten durch Kokartierung von 100 unterschiedlichen Proteinen an diesen Stellen auflösen. Dies zeigt, dass TIS sowohl das spektrale Limit als auch die Auflösungsleistung der traditionellen Fluoreszenzmikroskopie bei weitem übersteigt. Die Auflösungsleistung nimmt dabei mindestens proportional zur Anzahl der verwendeten Liganden zu, so dass generell gesagt werden kann, dass die Auflösungsleistung von TIS im Vergleich zu der eines traditionellen Fluoreszenzmikroskops ab etwa 20 bis 25 vermessenen Liganden bereits erheblich verbessert ist. Im vorliegenden Beispiel ist die Auflösungsleistung von ca. 40 nm ausreichend, um zwischen den drei Schichten der BL zu unterscheiden. Insgesamt ist der Informationsgehalt von TIS Datensätzen um Größenordnungen größer als der von großvolumigen ex vivo Molekularprofilen oder von Fluoreszenzbildern mit wenigen, inhaltsarmen Parametern. Dies wird unterstrichen durch viele 3D Toponom-Bilddaten, beispielsweise durch das Toponom von Lymphozyten aus peripherem Blut. Darüber hinaus können mit Hilfe von TIS grundsätzlich sogar Subdomänen von Proteinen charakterisiert werden, da der Abstand zwischen einer Fluoreszenzmarkierung (z. B. FITC) und der Bindungsstelle eines assoziierten Antikörpers vergleichsweise konstant ist und üblicherweise in der Größenordnung zwischen 3 nm und 5 nm liegt. Auch dieser relativ konstante Abstand begünstigt die hohe erzielbare Auflösungsleistung.

### TIS-Detektion der Lamina Densa der Haut als gigantisches Supermolekül

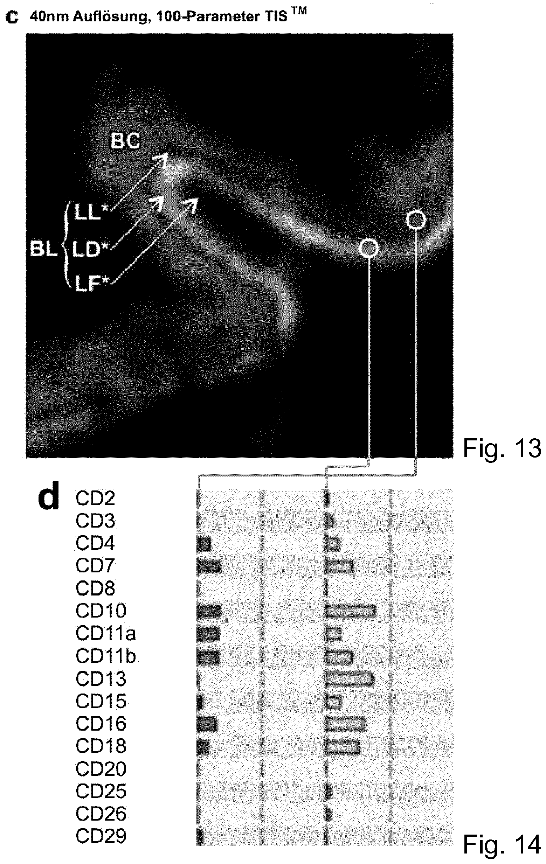

TIS-SIM Daten zeigen, dass die Lamina densa ein riesiges Supermolekül ist, welches einzigartig für diese BL-Schicht und inhärent unterschiedlich zur suprabasalen Epidermis und zur infrabasalen Dermis ist. Dies zeigt sich, weil nur Pixel, die zu Strukturen der Lamina densa gehören, in derselben Farbe hervorgehoben werden, wodurch das identische Proteinprofil entlang der gesamten lateralen Ausdehnung dieser bandartigen Struktur erscheint (Fig. 14 bis Fig. 16, grünes Profil). Somit sind die Pixel-Protein-Profile, welche die Lamina densa hervorheben, spezifisch und selektiv für exakt diese Struktur.

Fig. 11 bis Fig. 16 zeigen somit eine dermo-epitheliale Verbindung im menschlichen Gewebe: Visualisierung einer Basallamina densa als ein riesiges, stöchiometrisch gesteuertes Supermolekül. Ein Vergleich zwischen niedrig-auflösender, traditioneller Fluoreszenzmikroskopie (Fig. 12, b) mit einer 100-Parameter TIS-Abbildung (Fig. 13, c) und mit einer korrespondierenden Transmissionselektronenmikroskop-Aufnahme (TEM) (Fig. 11, a) zeigt, dass nur die funktionale Superauflösung der TIS-Mikroskopie (Fig. 13, c) die Basallamina densa (LD) als ein riesiges Supermolekül erfassen kann und dies bei einer Auflösung, die nahe an diejenige des TEM heranreicht: TIS kann die Lamina densa (DD) von der Lamina lucida (LL) und ebenso von der basalen Keratinozytenschicht (BC) unterscheiden, und der Lamina fibroreticularis (LF) (vgl. Fig. 11). Das Proteinprofil des entsprechenden, aus 100 Komponenten bestehenden Supermoleküls, welches in der LD exprimiert ist, ist in (Fig. 14 bis Fig. 16, Cluster 2, grün) zu sehen, welches sich von dem in der BC-Schicht (Fig. 14 bis Fig. 16, Cluster 1) exprimierten unterscheidet. Maßstabsbalken: 50 nm (Fig. 11, a). Zu bemerken ist, dass die Bilder in Fig. 12, b) und Fig. 13, c) gleichzeitig aus einem 5 Mikrometer (Mikron) starken, diagnostisch gefrorenen menschlichen Gewebeschnitt (Biopsiematerial) entnommen sind.

Darüber hinaus erstrecken sich die hervorgehobenen Pixel (Fig. 13, grünes Profil) über viele hundert Mikrometer (Mikron) und das Proteinprofil und die relative Häufigkeit der kokartierten Proteine verändert sich nicht entlang dieser Struktur. Dies zeigt, dass (i) eine stark gesteuerte Stöchiometrie der kokartierten Proteine und der assoziierten Kohlehydratstrukturen vorliegen muss und (ii) dass die ermittelte Struktur ein riesiges Supermolekül ist, das als ein spezifisches Merkmal der Lamina densa exprimiert wird. Dies hat viele wichtige Konsequenzen für das Verständnis des Biomolekülsystems in menschlichem Gewebe in vivo/in situ im Allgemeinen: (i) Die TIS-Technologie ist imstande, Supermoleküle innerhalb von Zellen und Geweben bei hoher funktionaler und struktureller Auflösung zu ermitteln; (ii) dieser technologische Fortschritt erlaubt es Forschern, praktisch alle existierenden Modi und Regeln topologischer und funktionaler Organisationen von Proteinnetzwerken aller menschlicher Proteine in großem Umfang sichtbar zu machen, und zwar über menschliche Gewebe und Erkrakungen hinweg durch sequenzielle Datenfelder von TIS-Hyperzyklen (wird anderweitig publiziert); (iii) der TIS-Ansatz kann mit Genomsequenzierung verknüpft sein. Somit kann die Genomanalyse mit der Analyse der dem Genom nachgelagerten operativen Ebene kombiniert sein, welche das Toponom ist, während das Toponom seine eigenen räumlichen Kodierungsregeln besitzt. Dies dient unmittelbar dem Verständnis der Wechselbeziehungen zwischen Genom- und ToponomStrukturen und -Funktionalitäten in denselben Geweben.

### Internationale TIS-Cluster als virtuelle Fabriken zur Dekodierung des Toponoms

Zu diesem Zweck können TIS-Cluster als virtuelle Fabriken sowohl innerhalb jeder gegebenen Einrichtung, aber auch als virtuelle Fabriken durch eine Verbindung von mehreren örtlich voneinander entfernten TIS-Robotern geschaffen werden. Dies wird einen schnellen Forschritt in der Dekodierung von Krankheitsmechanismen und grundlegender biologischer Vorgänge durch eine Verknüpfung spezialisierter Einrichtungen weltweit ermöglichen. Es wird das Konzept der Förderung und Integration verschiedener biologischer und anderer gewachsener wissenschaftlicher Kulturen, wie etwa der Mathematik, zur raschen Erlangung biologischer Erkenntnisse verfolgt. Die resultierende Kraft funktioneller Erkenntnisse und progressiven Verständnisses führt zur Entdeckung der spezifischen, chronische Krankheiten bestimmenden Mechanismen in den entsprechenden Geweben.

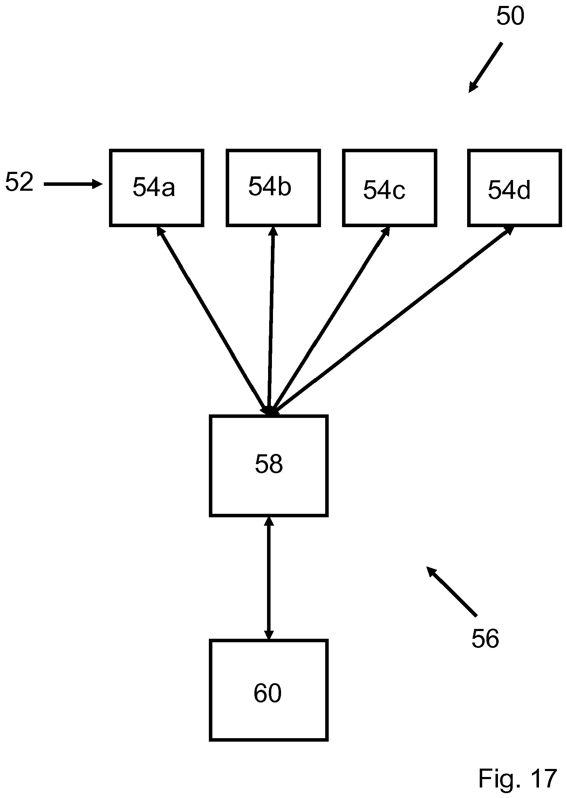

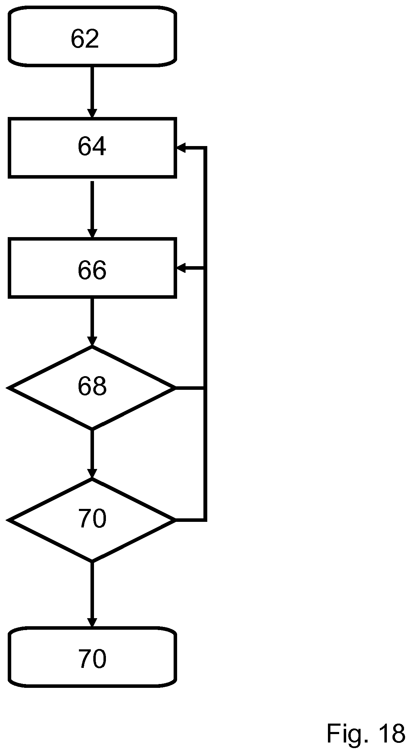

Fig. 17 und 18 zeigen zur weiteren Verdeutlichung eine beispielhafte Prinzipdarstellung einer als TIS-Cluster ausgebildeten Vorrichtung 50 zum Untersuchen von Zellproben sowie ein Beispiel eines Ablaufdiagramms eines Reverse-Imaging-Verfahrens, das mit Hilfe eines solchen TIS-Clusters 50 durchgeführt werden kann. Das TIS-Cluster 50 umfasst eine Einrichtung 52 zum Ermitteln von ortsaufgelösten kombinatorischen Bindungsmustern (KBM) mit Hilfe von Ligandenbibliotheken in wenigstens zwei unterschiedlichen Zellelementen wenigstens einer Zelle einer Zellprobe. Die Einrichtung 52 umfasst im gezeigten Ausführungsbeispiel vier Messeinrichtungen 54a-d, die ein Cluster bilden, wobei jeder Messeinrichtung 54a-d eine Ligandenbibliothek mit mindestens zwei Liganden zugeordnet ist. Die Messeinrichtungen 54a-d sind dazu ausgebildet, das Bindungsverhalten jedes Liganden aus der jeweiligen Ligandenbibliothek in der jeweiligen Zellprobe ortsaufgelöst zu ermitteln. Die Messeinrichtungen 54a-d sind zum Datenaustausch über ein Netzwerk 56 mit einer Datenbank 58 verbunden und speichern Bindungsdaten, die das ortsaufgelöste Bindungsverhalten jedes Liganden in der Zellprobe charakterisieren, in der Datenbank 58. Die Datenbank 58 ist über das Netzwerk 56 mit einer Recheneinrichtung 60 gekoppelt. Es ist zu betonen, dass das Netzwerk 56 grundsätzlich auch eine abweichende Netzwerktopolgie aufweisen kann. Ebenso kann die Datenbank 58 aus mehreren Teildatenbanken bestehen, die vorzugsweise über das Netzwerk 56 miteinander verbundenen sind.

Die Recheneinrichtung 60 ist ausgebildet, ortsaufgelöste kombinatorische Bindungsmuster (KBM) anhand der von den Messeinrichtungen 54a-d ermittelten und in der Datenbank 58 gespeicherten Bindungsdaten zu ermitteln und die ermittelten KBMs zu vergleichen, um Aussagen über das Vorliegen einer möglicherweise pathologischen Veränderung bei einer oder mehreren der in den Messeinrichtungen 54a-d vermessenen Zellproben tätigen zu können. Vorzugsweise steuert bzw. regelt die Recheneinrichtung 60 die Messeinrichtungen 54a-d derart, dass diese die für die jeweilige Fragestellung erforderlichen Bindungsstudien arbeitsteilig und/oder parallel und/oder jeweils in unterschiedlichen Zellelementen bzw. -kompartimenten durchführen. Die Messeinrichtungen 54a-d können aber grundsätzlich unabhängig voneinander über die Recheneinrichtung 60 gesteuert bzw. geregelt werden, um die jeweilige Fragestellung optimal klären zu können.

Die Messeinrichtungen 54a-d können dabei grundsätzlich kooperativ vorgehen, so dass jede Messeinrichtung 54a-d einen bestimmten Teil der Ligandenbibliothek verwendet. Alternativ oder zusätzlich können die Messeinrichtungen 54a-d jeweils die vollständige Ligandenbibliothek verwenden. Dies empfiehlt sich beispielsweise dann, wenn die jeweils vermessenen Zellproben aus unterschiedlichen Geweben bzw. Gewebeproben und/oder von unterschiedlichen Patienten stammen.

Sobald die Recheneinrichtung 60 anhand der in der Datenbank 58 zentral gespeicherten Bindungsdaten ein pathologisches kombinatorisches Bindungsmuster KBM ermittelt, beispielsweise ein für Brustkrebs spezifisches KBM, werden die Messeinrichtungen 54a-d entsprechend angesteuert bzw. geregelt, um die Frage zu klären, wo in der Zelle bzw. in welchem Zellelement die Ursache für das pathologische KBM liegt. Hierzu können die einzelnen Zellelemente der betrachteten Zellen schrittweise entgegen des biologischen Informationsflusses der Zellen vermessen werden, bis die zellbiologische Ursache für die krankhafte Veränderung ermittelt ist. Das für eine bestimmte Erkrankung als charakteristisch ermittelte KBM kann ebenfalls in der Datenbank 58 gespeichert und im Rahmen zukünftiger Messungen verwendet werden. Weiterhin kann die Datenbank 58 grundsätzlich auch zusätzliche externe Daten, beispielsweise Genomdaten enthalten, die bei der Beurteilung der ermittelten KBMs mit berücksichtigt werden. Da sich krankheitsspezifische KBMs in der Regel über etwa 6 bis 12 Liganden (Antikörper) eindeutig identifizieren lassen, können Zellproben, bei denen beispielsweise geprüft werden soll, ob Brustkrebs vorliegt oder nicht, zukünftig besonders schnell und zuverlässig vermessen werden.

Anhand von als krankheitsspezifisch erkannten KBMs können entsprechend krankheitsspezifisch kalibrierte Ligandenbibliotheken definiert und ebenfalls in der Datenbank 58 gespeichert werden, um die Messeinrichtungen 54a-d bei Bedarf mit den entsprechenden Liganden bestücken zu können. Beispielsweise können in der beschriebenen Weise vier Ligandenbibliotheken definiert werden, die jeweils spezifisch für Brustkrebs, Prostatakrebs, Lungenkrebs und Kolonkrebs sind. Anschließend können die vier Messeinrichtungen 54a-d jeweils mit einer dieser vier Ligandenbibliotheken versehen werden, um die Zellprobe(n) auf das Vorliegen der häufigsten ektodermalen Tumorarten zu untersuchen. Falls die Zellprobe(n) auf das Vorliegen immunologischer Tumore oder anderweitiger pathologischer Veränderungen untersucht werden soll(en), sind entsprechend ausgewählte Liganden bzw. Ligandenbibliotheken zu verwenden.

Insbesondere an der Zelloberfläche als krankheitsspezifisch erkannte KBMs können zudem in einem weiteren Schritt als Zielstrukturen zur Entwicklung von Medikamenten verwendet werden, da die als krankheitsspezifisch kolokalisiert bzw. antikolokalisiert erkannten Biomoleküle gut von außen zugänglich an der Oberfläche der betreffenden Zellen liegen. Beispielsweise können in an sich bekannter Weise spezifische Antikörper, insbesondere bi- oder multispezifische Antikörper gegen die in situ als krankhaft erkannten Zielstrukturen (Targets) hergestellt und als Medikament gegen die betreffende Krankheit bereitgestellt werden. Wie der Erfinder festgestellt hat, findet sich bei krankhaften Zellveränderungen immer ein für eine bestimmte Erkrankung charakteristischer abnormer Biomolekülkomplex an der Zelloberfläche, der damit als Target für Medikamente verwendet werden kann.

Weiterhin kann vorgesehen sein, dass die Messeinrichtung 54a das Bindungsverhalten der verbleibenden Liganden der Ligandenbibliothek vermisst, während die Messeinrichtungen 54b-d mit unterschiedlichen Ligandenbibliotheken bestückt werden. Beispielsweise können die Messeinrichtungen 54b-d mit einer Ligandenbibliothek bestückt werden, welche Liganden enthält, die charakteristisch für ein bezüglich des Informationsflusses der betreffenden Zelle stromauf liegendes Zellelement bzw. Zellkompartiment sind (Reverse-Imaging-Ansatz). So kann zum Beispiel eine Ligandenbibliothek verwendet werden, die Liganden enthält, die spezifisch für Vesikel sind. Ebenso kann vorgesehen sein, dass die Messeinrichtungen 54b-d mit unterschiedlichen Ligandenbibliotheken bestückt werden, wobei jede Ligandenbibliothek Liganden enthält, die jeweils spezifisch für unterschiedliche, stromauf des Informationsflusses der Zelle liegende Zellelemente sind. Beispielsweise kann die Messeinrichtung 54b mit spezifischen Liganden für Vesikel, die Messeinrichtung 54c mit spezifischen Liganden für Mitochondrien und die Messeinrichtung 54d mit spezifischen Liganden für das Zytoskelett bestückt werden. Ebenso kann eine der Messeinrichtungen 54a-d mit einer Ligandenbibliothek bestückt werden, die charakteristisch für ein nicht unmittelbar stromauf, sondern weiter stromaufliegendes Zellelement, beispielsweise für den Nucleolus, ist, wobei hierdurch die Logik bzw. Systematik des Auffindens krankheitsspezifischer intrazellulärer Ursachen durch das Reverse-Imaging-Verfahren zunächst durchbrochen würde, da möglicherweise für die jeweilige Fragestellung überflüssige bzw. nicht unmittelbar erforderliche Bindungsmuster ermittelt werden könnten.

Sobald mit Hilfe der Messeinrichtungen 54b-d ein weiteres charakteristisches KBM für ein betreffendes Zellelement ermittelt wurde, kann die entsprechende Ligandenbibliothek erneut angepasst werden, um bezüglich des biologischen Informationsflusses weiter stromauf liegende Zellelemente zu vermessen.

Mit Hilfe des TIS-Clusters 50 können grundsätzlich alle ungefähr 20000 Oberflächenmoleküle, die grundsätzlich in menschlichen oder tierischen Zellen vorkommen können, innerhalb von wenigen Woche vermessen werden. Zur Beschleunigung kann das TIS-Cluster 50 natürlich auch um weitere Messeinrichtungen bzw. um weitere Cluster erweitert werden. Sobald die Zelloberfläche vermessen ist, kann weiter in die Tiefe geforscht werden.

In dem in Fig. 18 gezeigten Ablaufdiagramm wird in Schritt 62 zunächst das in Fig. 17 gezeigte TIS-Cluster 50 bereitgestellt. In Schritt 64 werden die Messeinrichtungen 54a-d jeweils mit einer Ligandenbibliothek bestückt. Die Ligandenbibliothek enthält beispielsweise zwischen 20 und 100 Liganden, die an Biomoleküle binden, die spezifisch für ein bestimmtes Zellelement sind. Die erste verwendete Ligandenbibliothek umfasst wie bereits erwähnt vorzugsweise Liganden, die an Biomoleküle der Zellmembran binden. In Schritt 66 werden die Zellproben in repititiven Zyklen mit jeweils einem Liganden der Ligandenbibliothek beaufschlagt und das Bindungsverhalten des betreffenden Liganden ermittelt. Das Ermitteln des Bindungsverhaltens kann je nach Art und Menge des Liganden zusätzlich einen oder mehrere Waschschritte sowie gegebenenfalls einen oder mehrere Desaktivierungsschritte, beispielsweise physikalisches und/oder chemisches Bleichen einer Fluoreszenzmarkierung des Liganden nach erfolgter Bindungsstudie umfassen. Das ermittelte Bindungsverhalten wird in der Datenbank 58 gespeichert. Die Recheneinrichtung 60 ermittelt die KBMs für die bereits vermessenen Liganden und prüft, ob ein für eine pathologische Abweichung charakteristisches KBM vorliegt. Falls noch nicht alle Liganden der Ligandenbibliothek vermessen sind oder falls noch kein charakteristisches KBM ermittelt werden konnte, wird erneut Schritt 66 mit einem anderen Liganden ausgeführt. Falls ein krankheitsspezifisches KBM ermittelt wurde, wird in Schritt 70 geprüft, ob ein bezüglich des biologischen Informationsflusses stromauf liegendes Zellelement existiert. Falls ja, werden eine oder mehrere des Messeinrichtungen 54a-d mit einer an dieses stromauf liegende Zellelement angepassten Ligandenbibliothek versehen und das Verfahren in Schritt 64 mit den neuen Liganden weitergeführt. Falls das für die krankhafte Veränderung verantwortliche Zellelement identifiziert wurde oder falls keine weiteren stromauf liegenden Zellelemente mehr existieren, wird das Verfahren in Schritt 72 beendet.

### Behandlung chronischer Krankheiten. Unterliegt das Versagen einer Logik?

Die ständig zunehmende Ineffizienz bei der Behandlung chronischer Krankheiten - trotz eleganter und logischer molekularbiologischer Studien und nützlich angewandter Mathematik - hat in letzter Zeit zu eindringlichen Warnungen geführt (World Alzheimers Report 2011). Die Hauptfrage, die von ernstlich besorgten Wissenschaftlern und Autoren gestellt wird lautet "Was machen wir falsch?" und ähnliche Bedenken sind auch von anderen geäußert worden, während die desillusionierte Pharmaindustrie Produktionsstätten schließt und tausende von Angestellten entlässt. Überraschenderweise wurden entsprechende frühe Warnungen in einem Bericht mit dem Titel "The Fruits of Genomics" (Lehman Brothers und Mc Kinsey 2001) von der Wissenschaft völlig in Abrede gestellt, derweil der eigentliche Grund des Problems offensichtlich noch gar nicht bekannt ist, und viele Bedenken hinsichtlich der gegenwärtigen Entwicklung zu hohen Ausfallquoten von Medikamenten zur Behandlung von Krebserkrankungen und zu Fehlschlägen bei der Behandlung von Alzheimererkrankungen sind immer noch ohne klares Lösungskonzept. Offensichtlich sind die vielen eleganten, logischen molekular- und zellbiologischen Studien und Modellstudien mit ihren überzeugenden wissenschaftlichen Erklärungen gescheitert bei der Übertragung der entsprechenden Konzepte auf Alzheimer-Therapien und ähnliche Erfahrungen wurden im Bereich der Krebsforschung gemacht. Dies weist darauf hin, dass die Logik der aktuellen wissenschaftlichen Praxis mit ihren etablierten zellbiologischen und tierischen Modellen nicht mit der Logik der chronischen Krankheit als solcher übereinstimmt. Warum?

Ein Gedankenexperiment: Angesichts dessen, dass das riesige Supermolekül, das als spezifisches und selektives Charakteristikum der Lamina densa in der Haut ermittelt wurde (und somit in einem morphologisch intakten Gewebe vorhanden ist) (Fig. 11 bis Fig. 16): Wenn dieser aufgezeigte Satz an 100 unterschiedlichen Biomolekülen und die spezifische Stöchiometrie dieser Moleküle innerhalb dieser Struktur nicht bekannt wäre, sondern wenn nur umfangreiche ex vivo Expressionsprofile der Haut (aus Gewebehomogenaten) bekannt wären, ist es dann möglich aus diesen ex vivo Profilen die genaue Zusammensetzung und Stöchiometrie dieser Lamina densaspezifischer Supermoleküle vorherzusagen und gleichzeitig vorherzusagen, dass andere Strukturen in der Umgebung (Lamina fibroreticularis, Lamina lucida, Basalkeratinozyten etc.) Fig. 11 bis Fig. 16) dieses nicht exprimieren? Die Antwort wird natürlich höchst wahrscheinlich negativ ausfallen. Aber nehmen wir einmal weiter an, diese erwähnten Supermoleküle seien ein spezifisches Merkmal eines vorliegenden Krebses in situ, und weiter, dass dieses Merkmal genau die Struktur sei, auf die ein Medikament selektiv gerichtet ist, dann ist es klar, dass das entsprechende selektive Medikament durch einen rationalen Ansatz auf der Grundlage von ex vivo Profilierung oder ex vivo Modellen nie gefunden würde. Dies liegt einfach an der Tatsache, dass der Informationsgehalt der spezifischen Zusammensetzung und Topologie der Supermoleküle in Geweben um Größenordnungen größer ist als der von ex vivo Expressionsdaten und daher durch ex vivo Expressionsprofilierung oder gegenwärtige zellbiologische oder andere Modelle nicht vorhergesagt werden kann.

### Toponomics: Dekodierung und Behandlung von Krankheiten im Kontext der Histologie

Eine interessante Frage lautet: Warum hat die Systembiologie den traditionellen Weg verlassen bzw. ihn von Anfang an nicht einmal beachtet, einen vom Prinzip der Histologie bestimmten Weg nämlich, der von seinem Vorreiter Marcello Malpighi (1628 - 1694) begründet wurde und der die Medizin zu der erfolgreichen, auf Anatomie basierenden Disziplin gemacht hat. Warum kehrte man sich in der modernen molekularen Systembiologie ab von der Histologie als der strukturellen Grundlage und dem historisch erfolgreichen Bereich der Diagnostik und der Forschung in der Humanmedizin bzw. betrachtete sie nie als wichtig für das Verständnis von Krankheiten. Eine naheliegende Antwort scheint die weithin anerkannte, aber nicht unkontroverse Ansicht zu sein, enorm große ex vivo Datensätze, wie Genomdaten oder ex vivo Expressionsprofil-Datensätze, könnten genügend und sogar mehr funktionelle Erkenntnisse als jede strukturgebundene Information in Geweben liefern. Es verwundert nicht, dass die sich ergebenden Datensätze, die keine Beziehung zu subzellulären funktionellen Topologien hatten, so groß wurden, dass Sekundärtechnologien und Algorithmen zu deren Betrachtung und Interpretation entwickelt werden mussten usw.: Dieses System wurde selbstbezüglich, indem es eine neue medizinische Forschung auf der Grundlage von ex vivo Daten betrieb und so die Auseinanderentwicklung von traditioneller mikroskopischer Anatomie und den neuen Ex-Vivo-Disziplinen beschleunigte. Aber ist eine Zelle oder ein Gewebe nicht ein großer Apparat zur molekularen Musterbildung mit klaren topologischen Funktionsregeln zur Kodierung von Funktionalitäten? Müssen wir diese topologisch basierten Modi und Regeln nicht in allen Einzelheiten verstehen, um die Achillesferse chronischer Krankheiten zu finden? Und können diese grundlegenden Prinzipien überhaupt durch eine Reduzierung der Zelle auf eine große Ansammlung von Biomolekülen verstanden werden? Toponomics wurde auf eigenen Wegen unabhängig von umfangreichen ex vivo Technologien entwickelt und kann dazu beitragen, diese Auseinanderentwicklung rückgängig zu machen, indem sie molekulare Systeme als funktionale Einheiten eines morphologisch intakten Gewebes betrachtet und so die subzelluläre Topologie von Proteinnetzwerkkodizes in das großangelegte Expressionsprofiling zu integrieren. Die Toponomics-Technologie gründete von Beginn ihrer Erfindung an gänzlich auf Histologie und blickt seither auf eine 22-jährige Tradition entsprechender Molekularhistologie zurück. Diese Technologie baut auf einer Stärke auf, die ihren Grund in der Fähigkeit zur Identifizierung krankheitspezifischer supramolekularer Merkmale und Ereignisse ohne a priori mechanistisches oder molekulares Wissen hat. Sie ist weitgehend hypothesefrei. Hierfür ist eine hohe subzelluläre Auflösung, die durch extreme dimensional unbeschränkte PCMDs generiert wird, unbedingt erforderlich (Fig. 11 bis Fig. 16). Der gegenwärtige Status innerhalb des humanen Toponom-Projekts erlaubt es Forschern, systematisch große TIS-Cluster als weltweite virtuelle Werkstätten zur Dekodierung kompletter Toponome menschlicher Proteine zu betreiben und diese Cluster mit Genomsequenzierung zu kombinieren.

Fig. 6 bis Fig. 10 zeigen eine Prinzipdarstellung eines Untersuchungsverfahrens, bei welchem KBMs in einer Zellprobe anhand des Bindungsverhaltens zahlreicher einzelner Liganden, die in einer Ligandenbibliothek zusammengefasst sind, ermittelt und räumlich dargestellt werden. Das beste Grundprinzip, das für diese Aufgabe verwendet werden kann, ist Fluoreszenzmikroskopie. Um jedoch die hochdimensionale kombinatorische Molekularorganisation des Toponoms in einer und derselben subzellulären Struktur zu erfassen, sollten viel mehr Proteine/Biomoleküle in einer Zelle oder in einem Gewebe gleichzeitig kokartiert werden als Wellenlängen innerhalb des Spektrums sichtbaren Lichts zur Verfügung stehen (zwischen ca. 300 und 700 nm). Wie lässt sich also diese Beschränkung überwinden? Die Antwort lieferte die Toponomtheorie der dimensional-unbeschränkten molekularen Bildgebung. Diese Theorie basiert auf großen Sonden bzw. Ligandenbibliotheken (20; 50; 100; 1,000 usw. Liganden), in denen jede Sonde, die spezifisch an eine bestimmte Grippe oder rein bestimmtes Biomolekül in situ bindet, mit wenigstens einem Farbstoff bzw. Fluoreszenzfarbstoff gekoppelt ist, beispielsweise Fluoresceinisothiocyanat (FITC), CY-3, CY-5, Rhodamin und dergleichen. Mehr als ein Farbstoff pro Zyklus ist ebenfalls möglich. Das Prinzip schließt weiterhin ein, dass ein eingehauster Roboter automatische und vorprogrammierte, sich wiederholende Zyklen von (i) Sonden-Farbstoff Inkubation auf dem Objekttisch eines Epifluoreszenzmikroskops; (ii) Abbilden des sich ergebenden Signals; und (iii) Deaktivierung dieses Signals mittels mildem Fluoreszenzbleichen (oder Deaktivierung unter Verwendung eines geeigneten chemischen Agens) ausführt. Fig. 6 bis Fig. 10 illustrieren dieses Prinzip von der zyklischen Datenerfassung bis hin zur 3D Toponom-Kartierung. Diese Theorie auf der Grundlage der sogenannten "Venedig"- und der "Sonnenlicht"-Hypothese einer dimensions- und parameter-unlimitierten molekularen Bildgebung ist experimentell verifiziert worden, nachdem mehrere Generationen entsprechender Roboter entwickelt worden waren (Multiepitop-Liganden-Kartographie, multi-epitope-ligand-cartography, MELC, und Toponom-Abbildungssysteme, toponome imaging systems, TIS) die gemeinsam als "imaging cyclers" bezeichnet werden.

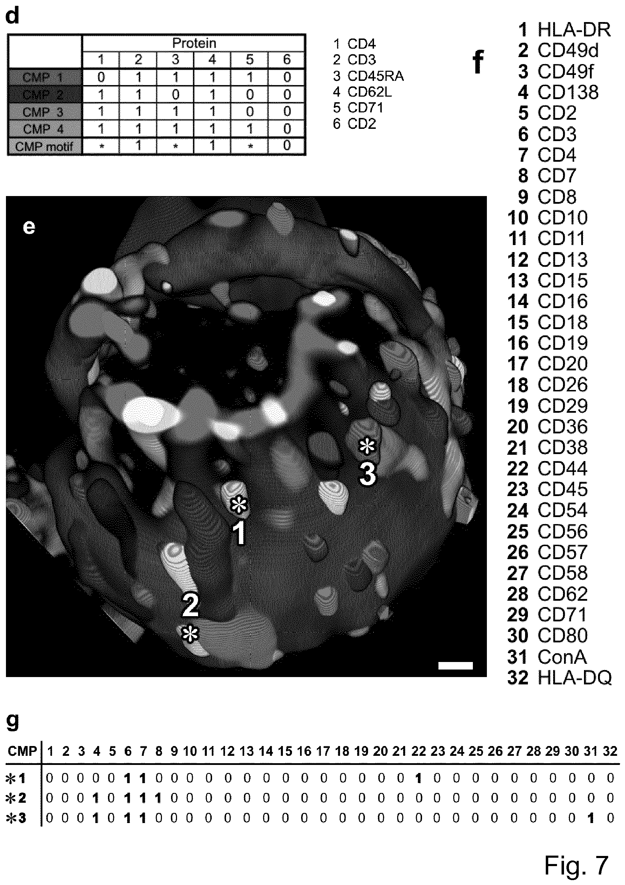

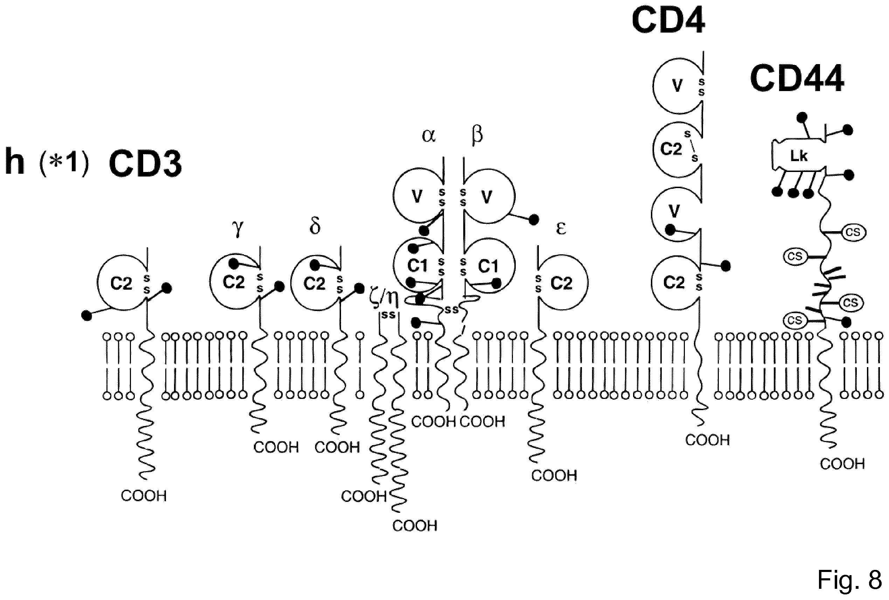

Ein Beispiel für eine zyklische TIS-Methode an mononuklearen Lymphozyten des peripheren Bluts (PBML) zur Gewinnung einer zweidimensionalen (a - d) und einer dreidimensionalen Toponomkarte von Multi-Protein Clustern der Zelloberfläche (e) einschließlich eines Beispiels für deren funktionale Annotation (g-j) ist in Fig. 6 bis Fig. 10 dargestellt. (a) Insgesamt wurden 9 TIS Zyklen durchlaufen mit 2 mit einem Farbstoff konjugierten Sonden/Antikörpern pro Zyklus (FITC, PE als Farbstoffe), um 18 verschiedene Zelloberflächenproteine in einem fixierten Zellmuster zu markieren. Die gekennzeichneten Proteine sind rechts von der (b, vertikalen Liste) spezifiziert. (A, A', A"): Die gleiche Sondenbibliothek wurde verwendet, um drei sich wiederholende TIS-Zyklen an dem identischen Zellmuster (A bis A") durchzuführen, während die Abfolge der Zyklen unverändert blieb (sogenannte wiederholte Vorwärts-TIS-Durchläufe, "repetitive forward TIS runs"). Die Spezifität der Proteinmarkierung (protein tagging) an jeder Stelle und die Abwesenheit von sterischer Behinderung der Antikörperbindung während des TIS-Vorgangs wird verifiziert durch (i) die horizontale Ausrichtung jedes Signals (A bis A") und (ii) Quantifizierung korrelierter Signalintensitäten des sich ergebenden Pixel-Datensatzes mittels mathematischer Methoden: Es ist festzustellen - durch einen Vergleich von A, A', und A" (repetitive forward TIS runs) - dass die Signalorte von A bis A" identisch sind (horizontales Bildfeld), während die Signalintensitäten aufgrund von progressiver Sättigung der entsprechenden Antikörper-bindenden Epitope abnehmen. Solche Sätze routinemäßiger TIS-Bewertungs-Vorgänge, einschließend wiederholte vorwärts, invertierte und permutierte TIS-Läufe, liefern weitere Beweise für die quantitative Präzision von TIS. Es ist Teil des sogenannten logischen high-end Kalibrierungsprozesses von TIS. (b) Illustration des Prozesses der Toponom-Kartierung durch die Darstellung zweier Zellen (b, vertikale Zeilen 1 und 2; Zellen vergrößert und ausgerichtet in b'). Überlagerung und Ausrichtung wird verwendet, um Schwellen für jedes Fluoreszenzsignal zu setzen (Fig. 7, d), Experten-basiert oder automatisiert), um Multi-Protein-Cluster (KBMs/CMPs) Bereiche und das entsprechende CMP-Motiv in (c) zu identifizieren, Farbdekodierungsliste in Fig. 7, d). Fig. 7, e) zeigt die 3-D Toponomkarte eines einzigen CD4-PBML, gewonnen durch optische Sektionierung während der TIS-Bildgebung: 32 TIS-Zyklen wurden auf jeder von 20 verschiedenen optischen Ebenen über die Zelle durchgeführt (Sonden-/Antikörperbibliothek in Fig. 7, f). Fig. 7, g) zeigt, wie CMP 1-3 aus mehreren tausend CMPs pro dieser Zelle insgesamt extrahiert werden, um darzustellen, welche Proteine (g, oberste Zeile) unterschiedlich assoziiert sind als einzelne Protein Clusters (CMPs) bzw. kombinatorische Bindungsmuster (KBM) auf der Zelloberfläche (Fig. 7, e), Asteriske 1 bis 3 entsprechen CMP 1 bis 3 in Fig. 7, g). Fig. 8 bis Fig. 10 (h-j) veranschaulichen die durch TIS aufgezeigten supramolekularen Verbände (CMPs/KBMs) in Fig. 7, e), Asteriske 1 - 3 durch Verwendung eines räumlichen Modells der einzelnen kokartierten Transmembranproteine (Fig. 8 bis Fig. 10 bzw. h-j: entsprechend zu jeweiligen Asterisken 1 bis 3 in Fig. 7, e). Die entsprechende Zelloberflächenstruktur in h bis j der einzelnen Proteine wurde mit bekannten Techniken rekonstruiert. Zu bemerken ist, dass nicht auf Schwellwert basierte TIS in Fig. 11 bis Fig. 16 veranschaulicht ist.

Das ursprünglich von George Kingsley Zipf beschriebene Gesetz wurde ursprünglich angewandt, um die Gesetzmäßigkeiten der Häufigkeit von Wörtern in menschlichen Sprachen zu analysieren. Dabei ergibt das Produkt aus Rang und Häufigkeit von Wörtern (Wörter = Kombination von Buchstaben als kleinste semantische Einheit in Sprachsystemen) eine immer annähernd gleiche Konstante. In hochdimensionalen Toponom-Datensätzen ergibt sich eine ähnliche Konstante, wenn man die Protein-Cluster (= spezifische Protein-Kombinationen an einem bestimmten Ort als kleinste semantische Einheit des Toponoms), d. h. räumlich (topologisch) genau definierte Zusammenlagerungen bestimmter Proteine in der Zelle misst. Die für einen bestimmten Zelltyp und seine Funktionszustände hoch spezifischen Cluster kann man mit der oben beschriebenen Toponom-Technologie direkt sichtbar machen.

Die biologische Bewertung des oben genannten Prinzips wurde in mehreren Studien geliefert, die zeigten, dass diese Technologie Schlüsselprobleme in Biologie und Medizin lösen kann. Beispielsweise wurde in einer frühen Anwendung gezeigt, dass Endothelzellen in den endomysialen Tubus während der Muskelregeneration bei Menschen eindringen, wo sie transdifferenzieren und myogene Stammzellen erzeugen, die das gerissene Muskelsegment in dem durch die Basallaminastrukturen ausgebildeten endomysialen Tubus regenerieren.

Diese Entdeckung der Transdifferenzierung von adulten Endothelzellen zur Ausbildung von Muskelstammzellen innerhalb eines menschlichen Gewebes wurde durch mehrere experimentelle Studien bestätigt und wurde als neuartiges Zelltherapiemodell zur Behandlung neuro-muskulärer Krankheiten weiterentwickelt. In ähnlicher Weise wurde ein neues Zielprotein in amyotrophischer lateraler Sklerose durch hierarchische Proteinnetzwerkanalyse entdeckt - eine Erkenntnis, die durch ein K.O.-Maus-Modell bestätigt wurde. Des Weiteren hat dieses ein Leitzielprotein in Tumorzellen entdeckt, welches die Zellpolarisierung/Metastase steuert, und (iv) ein neues Zielprotein gefunden, welches chronische neuropathische Schmerzen steuert, eine Erkenntnis, die durch ein unabhängiges K.O.-Maus-Modell bestätigt wurde. Aktuelle Forschung zeigte überraschenderweise, dass Toponom Fingerprinting von peripheren menschlicher Blutlymphozyten eine progressive Nervenkrankheit fünf Jahre vor ihrem klinischen Ausbruch (Schubert W, persönliche Kommunikation) detektieren kann und dass eine monogenetische Krankheit durch Umwandlung des Genotypspezifischen Toponomcodes in einen normalen durch Verwendung einer geringen nicht-toxischen Medikation (M. Ruonala et al., persönliche Kommunikation) erfolgreich behandelt werden kann. Zusammengenommen zeigen diese technologischen, biologischen und klinischen Bewertungen, dass MELC/TIS eine ausgereifte Technologie ist, die imstande ist, subzelluläre Krankheitsmechanismen zu dekodieren, neue Zielstrukturen für Medikamente aufzudecken und auch neue effiziente Medikamente gegen chronische Erkrankungen, Tumore etc. zu entwickeln, und zwar direkt an den Zielorten der Krankheit im menschlichen Gewebe (Biopsien und/oder chirurgisches Material).

Die in den Unterlagen angegebenen Parameterwerte zur Definition von Prozess- und Messbedingungen für die Charakterisierung von spezifischen Eigenschaften des Erfindungsgegenstands sind auch im Rahmen von Abweichungen - beispielsweise aufgrund von Messfehlern, Systemfehlern, Einwaagefehlern, DIN-Toleranzen und dergleichen - als vom Rahmen der Erfindung mitumfasst anzusehen.

## Patentansprüche

1. Verfahren zum Untersuchen einer Zellprobe, umfassend zumindest die Schritte:
- Ermitteln von ortsaufgelösten kombinatorischen Bindungsmustern (KBM) mit Hilfe einer wenigstens zwei Liganden umfassenden Ligandenbibliothek in wenigstens zwei Zellelementen aus der Gruppe Gruppe DNA, RNA, Transkriptionsfaktor, Nukleolus, Nucleus, Endoplasmatisches Reticulum, CIS Golgi Vesikel, Golgi-Apparat, Vesikel, Trans-Golgi-Netzwerk, Zellmembran, Cytoskelett, Mitochondrien, Lysosom, Peroxisomen, Vesikel, Ribosomen, Mikrotubuli und Zentriolen wenigstens einer Zelle der Zellprobe, wobei die KBMs in wenigstens zwei Zellelementen, welche sich zumindest bezüglich ihrer Reihenfolge entlang des biologischen Informationsflusses der Zelle unterscheiden, entgegen der Reihenfolge des biologischen Informationsflusses der Zelle ermittelt werden;
- Vergleichen der ermittelten KBMs; und
- Ableiten anhand des Vergleichs der KBMs, ob zumindest eines der betrachteten Zellelemente pathologisch verändert ist.

2. Verfahren nach Anspruch 1, bei welchem eine Ligandenbibliothek verwendet wird, die wenigstens einen Zellelement-spezifischen Liganden und/oder wenigstens einen Liganden aus der Gruppe unspezifische Liganden, spezifische Liganden, markierte Liganden, monoklonale Antikörper, polyklonale Antikörper, rekombinante Antikörper, bispezifische Antikörper, Lectine, Nukleinsäuren, Nukleinsäuresequenzen, Peptide, Aptamere und Toxine umfasst.

3. Verfahren nach Anspruch 1 oder 2, bei welchem eine Information bereitgestellt und/oder ermittelt wird, ob die Zellprobe pathologisch verändert ist, und/oder bei welchem anhand des Vergleichs der KBMs eine Zielstruktur für die Entwicklung eines Medikaments zur Behandlung eines Patienten, der unter einer durch eine pathologische Veränderung der betrachteten Zellprobe charakterisierten Erkrankung leidet, ermittelt wird.

4. Verfahren nach Anspruch 1 bis 3, bei welchem in wenigstens einem der Zellelemente erneut KBMs mit Hilfe einer um wenigstens einen weiteren Liganden erweiterten Ligandenbibliothek ermittelt werden, wenn das wenigstens eine Zellelement als pathologisch verändert eingestuft wurde.

5. Verfahren nach einem der Ansprüche 1 bis 4, bei welchem wenigstens ein weiteres KBM mit Hilfe einer wenigstens zwei Liganden umfassenden Ligandenbibliothek in wenigstens einem weiteren Zellelement wenigstens einer Zelle der Zellprobe ermittelt und mit wenigsten einem der bereits ermittelten KBMs verglichen wird, wenn ein Unterschied der bereits miteinander verglichenen KBMs eine vorbestimmte Unterscheidungsschwelle nicht überschreitet.

6. Verfahren nach einem der Ansprüche 1 bis 5, bei welchem die KBMs in einer einzigen Zelle der Zellprobe und/oder in einem einzigen Gewebeschnitt und/oder in mehreren Zellen derselben Zellprobe und/oder in mehreren Zellen unterschiedlicher Zellproben ermittelt werden und/oder bei welchem wenigstens eines der KBMs mit Hilfe eines Fluoreszenzverfahrens, insbesondere eines multidimensionalen Fluoreszenzmikroskopieverfahrens, und/oder mit Hilfe eines MELK-Roboters und/oder mit Hilfe eines Toponome-Imaging-Systems ermittelt wird.

7. Verfahren nach einem der Ansprüche 1 bis 6, bei welchem wenigstens eines der ermittelten KBMs als Toponomdatensatz in einer Datenbank einer Recheneinrichtung gespeichert wird und/oder bei welchem zum Vergleichen der KBMs Toponomdatensätze der entsprechenden KBMs mit Hilfe der Recheneinrichtung bereitgestellt und miteinander verglichen werden.

## Claims

1. Method for examining a cell sample including at least the steps of:
- determining spatially resolved combinatorial binding patterns (KBM) with the aid of a ligand library including at least two ligands in at least two cell elements from the group of DNA, RNA, transcription factor, nucleolus, nucleus, endoplasmatic reticulum, CIS Golgi vesicles, Golgi apparatus, vesicles, trans-Golgi network, cell membrane, cytoskeleton, mitochondria, lysosome, peroxisomes, vesicles, ribosomes, microtubules and centrioles of at least one cell of the cell sample, wherein the KBMs are determined in at least two cell elements, which differ at least with respect to their order along the biological information flow of the cell, opposite to the order of the biological information flow of the cell;
- comparing the determined KBMs; and
- inferring if at least one of the considered cell elements is pathologically altered based on the comparison of the KBMs.

2. Method according to claim 1, in which a ligand library is used, which includes at least one ligand specific to cell element and/or at least one ligand from the group of unspecific ligands, specific ligands, labeled ligands, monoclonal antibodies, polyclonal antibodies, recombinant antibodies, bispecific antibodies, lectins, nucleic acids, nucleic acid sequences, peptides, aptamers and toxins.

3. Method according to claim 1 or 2, in which information is provided and/or determined if the cell sample is pathologically altered and/or in which a target structure for development of a medicament for treatment of a patient suffering a disease **characterized by** a pathologic alteration of the considered cell sample is determined based on the comparison of the KBMs.

4. Method according to claim 1 to 3, in which KBMs are again determined in at least one of the cell elements with the aid of a ligand library extended by at least one further ligand if the at least one cell element was classified as pathologically altered.

5. Method according to any one of claims 1 to 4, in which at least one further KBM is determined with the aid of a ligand library including at least two ligands in at least one further cell element of at least one cell of the cell sample and compared to at least one of the already determined KBMs if a difference of the KBMs already compared to each other does not exceed a predetermined differentiation threshold.

6. Method according to any one of claims 1 to 5, in which the KBMs are determined in a single cell of the cell sample and/or in a single tissue section and/or in multiple cells of the same cell sample and/or in multiple cells of different cell samples and/or in which at least one of the KBMs is determined with the aid of a fluorescence method, in particular a multi-dimensional fluorescence microscopy method, and/or with the aid of a MELK robot and/or with the aid of a toponome imaging system.

7. Method according to any one of claims 1 to 6, in which at least one of the determined KBMs is stored in a database of a computing device as a toponome dataset and/or in which toponome datasets of the corresponding KBMs are provided and compared to each other with the aid of the computing device for comparing the KBMs.

## Revendications

1. Procédé d'étude d'un échantillon de cellules, comprenant au moins les étapes, consistant à :
- déterminer des schémas de liaison combinatoires, à résolution spatiale (SLC), à l'aide d'une bibliothèque de ligands, comprenant au moins deux ligands, dans au moins deux éléments cellulaires dans le groupe se composant du groupe de l'ADN, de l'ARN, du facteur de transcription, du nucléole, du noyau, du réticulum endoplasmique, de la vésicule golgienne CIS, de l'appareil de Golgi, des vésicules, du réseau transgolgien, de la membrane cellulaire, du cytosquelette, des mitochondries, du lysosome, des peroxysomes, des vésicules, des ribosomes, des microtubules et des centrioles, au moins d'une cellule de l'échantillon de cellules, les SLC étant déterminés, dans au moins deux éléments cellulaires, lesquels se différencient, au moins en ce qui concerne leur séquence le long du flux d'informations biologiques de la cellule, contrairement à la séquence du flux d'informations biologiques de la cellule ;
- comparer les SLC déterminés et
- déduire, à l'aide de la comparaison des SLC, si au moins l'un des éléments cellulaires considérés est modifié pathologiquement.

2. Procédé selon la revendication 1, pour lequel une bibliothèque de ligands est utilisée, qui comprend au moins un ligand spécifique de l'élément cellulaire et / ou au moins un ligand dans le groupe se composant de ligands non spécifiques, des ligands spécifiques, des ligands marqués, des anticorps monoclonaux, des anticorps polyclonaux, des anticorps recombinants, des anticorps bispécifiques, des lectines, des acides nucléiques, des séquences d'acide nucléique, des peptides, des aptamères et des toxines.

3. Procédé selon la revendication 1 ou 2, pour lequel une information est mise à disposition et / ou déterminée, sur la question qui est de savoir si l'échantillon de cellules est modifié pathologiquement et / ou pour lequel une structure cible est déterminée, à l'aide de la comparaison des SLC, pour la mise au point d'un médicament destiné au traitement d'un patient, qui souffre d'une maladie **caractérisée par** une modification pathologique de l'échantillon de cellules considéré.

4. Procédé selon la revendication 1 à 3, pour lequel des SLC sont à nouveau déterminés, dans au moins l'un des éléments cellulaires, à l'aide d'une bibliothèque de ligands, étendue d'au moins un autre ligand, lorsque le au moins un élément cellulaire a été classé comme pathologiquement modifié.

5. Procédé selon l'une des revendications 1 à 4, pour lequel au moins un autre SLC est déterminé à l'aide d'une bibliothèque de ligands, comprenant au moins deux ligands, dans au moins un autre élément cellulaire d'au moins une cellule de l'échantillon de cellules et est comparé avec au moins l'un des SLC, déjà déterminés, lorsqu'une différence des SLC, déjà comparés entre eux, n'excède pas un seuil différentiel prédéterminé.

6. Procédé selon l'une des revendications 1 à 5, pour lequel les SLC sont déterminés dans une cellule unique de l'échantillon de cellules et / ou dans une coupe de tissu unique et / ou dans plusieurs cellules du même échantillon de cellules et / ou dans plusieurs cellules d'échantillons différents de cellules et / ou pour lequel au moins l'un des SLC est déterminé à l'aide d'un procédé de fluorescence, en particulier d'un procédé de microscopie en fluorescence multidimensionnelle et / ou à l'aide d'un robot de Melk et / ou à l'aide d'un système d'imagerie toponymique.

7. Procédé selon l'une des revendications 1 à 6, pour lequel au moins l'un des SLC déterminés est enregistré comme jeu de données toponymiques dans une base de données d'un dispositif de calcul et / ou pour lequel, en vue de la comparaison des SLC, des jeux de données toponymiques des SLC correspondants sont mis à disposition à l'aide du dispositif de calcul et comparés entre eux.
